# EUROPEAN PATENT APPLICATION

(11) **EP 1 795 526 A1**
(43) Date of publication of application: **13.06.2007**
(21) Application number: 05785892.0
(22) Date of filing: 13.09.2005
(51) Int. Cl.: C07D 211/58, A61K 31/4468, A61K 31/4525, A61K 31/4535, A61P 25/02, A61P 25/04, C07D 405/12, C07D 409/12

(54) **N-SUBSTITUTED N-(4-PIPERIDINYL)AMIDE DERIVATIVE**

(30) Priority: 14.09.2004 JP 2004267238
(71) Applicant: Nippon Chemiphar Co., Ltd., Chiyoda-ku Tokyo 1010032 (JP)
(72) Inventor: TAKAHASHI, Toshihiro, Saitama,3410021 (JP); ENDO, Tsuyoshi, Tokyo, 1570077 (JP); USHIODA, Masatoshi, 1830042 (JP); KOBAYASHI, Kunio, 3410018 (JP); YAMAKAWA, Tomio, 2770884 (JP); SHIKA, Kiichi, 3440041 (JP); KAWASAKI, Toru, 124-0013, Tokyo (JP); IMAI, Toshiyasu, 2740072 (JP)
(74) Representative: Albrecht, Thomas
(86) International application number: PCT/JP2005/017217
(87) International publication number: WO 2006/030931

(57) **Abstract**

An N-substituted N-(4-piperininyl)amide derivative represented by the following formula (I): (wherein R¹ represents C₁₋₆ alkyl, furyl, etc.; R² represents amino, acetylamino, ureido, etc.; R³ represents hydrogen, C₂₋₈ alkoxycarbonyl, etc.; R⁴ represents optionally substituted phenyl, etc.; R⁵ represents hydroxyl, benzyloxy, etc.; R⁶ represents hydrogen or methyl; and m is 1 or 2) or a salt of the derivative, and an analgesic containing the derivative or salt.

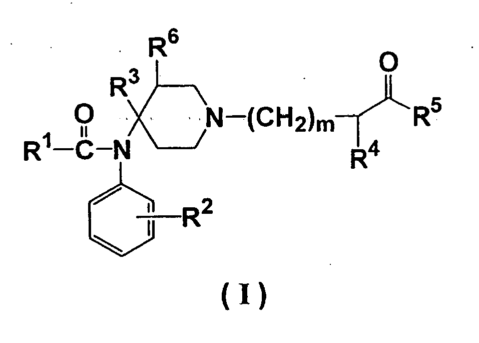

## Description

### [FIELD OF THE INVENTION]

The present invention relates to an N-substituted-N-(4-piperidinyl)amide derivative or a salt thereof, and an analgesic containing the same as an effective ingredient.

### [BACKGROUND OF THE INVENTION]

A number of N-substituted N-(4-piperidinyl)amide derivatives are reported in a number of publications such as JP 51-115478A (Patent publication 1), JP 53-149980A (Patent publication'2), JP 2-292279A (Patent publication 3), JP 2-300167A (Patent publication 4) and Zhongguo Yaoke Daxue Xuebao 1993, 24, p 257-263 (Non-patent publication 1). It is known that these derivatives have an analgesic effect and a narcotic effect.

Patent publication 1 describes that N-substituted N-(4-piperidinyl)amide derivatives of the following formula (A) have an analgesic effect:

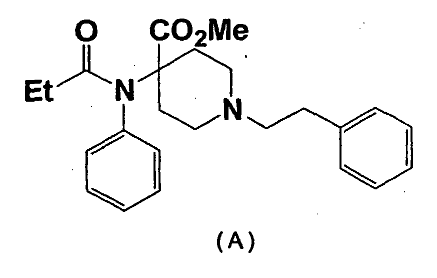

Patent publication 4 describes that N-substituted N-(4-piperidinyl)amide derivatives of the following formula (B) have an analgesic effect:

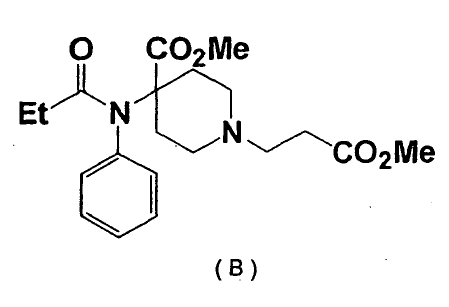

Non-patent publication 1 describes that N-substituted N-(4-piperidinyl)amide derivatives of the following formula (C) were studied with respect to narcotic effect:

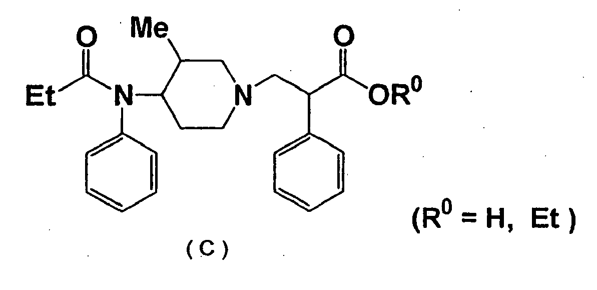

Fentanyl which is a representative N-substituted-N-(4-piperidinyl)amide derivative is practically employed in clinical use as auxiliary narcotic and analgesic. The analgesic effect of the N-substituted N-(4-piperidinyl)-amide derivatives are considered to be mediated agonist action to µ-receptor, which is one of opioid receptors.

As the µ-opioid receptor agonist, morphine as well as the N-substituted N-(4-piperidinyl)amide derivatives such as Fentanyl are well known. However, since these drugs show adverse effects such as drug dependence, bradycardia, respiratory depression, and inhibition of digestive tube motility, it is demanded to provide a new analgesic showing reduced adverse effects.

Until now, it has been considered that the analgesic effect is centrally mediated by the µ-receptor. However, C. Stein, Anesth. Analg., 1993, 76, 182-191 (Non-patent publication 2), C. Stein, The New England Journal of Medicine, 1995, 332, 1685-1690 (Non-patent publication 3), and A. Herz, Progress in Brain Res., 1996, 110, 95-104 (Non-patent publication 4) have recently reported that there is a µ-receptor in the terminal of peripheral sensory nerve and that there is an analgesic mechanism via this µ-redeptor.

Accordingly, the present inventors assumed that a drug which shows an analgesic effect by selectively mediated by peripheral µ-receptor would be an analgesic showing no adverse effects such as drug dependence caused by central action, and disclosed N-phenyl-N-(4-piperidinyl)-amide derivatives having the following formula (D): 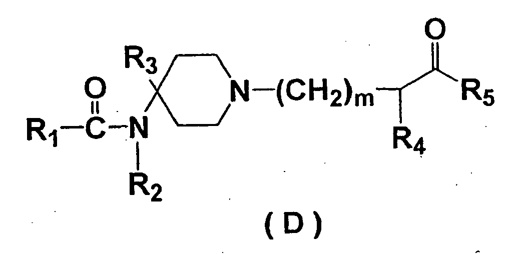 (in which R₁ represents an alkyl group having 1 to 6 carbon atoms, etc.; each of R₂ and R₄ represents a phenyl group which optionally has a substituent, R₃ represents a hydrogen atom, an alkoxycarbonyl group having 2 to 8 carbon atoms, or a methyl group substituted with an alkoxy group having 1 to 6 carbon atoms; R₅ represents a residue of amino acid such as glycine, alanine, leucine, phenylalanine, etc., and m represents 1 or 2) in a patent application (WO 03/082819, Patent publication 5).

In the above-mentioned application, there is also disclosed an intermediate compound for preparing a compound of the formula (D), which corresponds to a compound having a hydroxyl group as R₅ and is represented by the following formula (E): 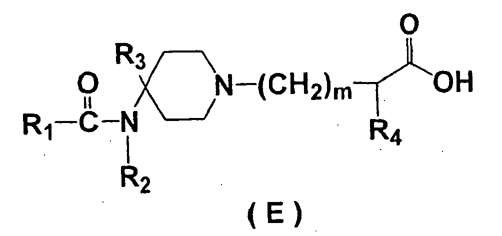 (in which each of R₁, R₂, R₃, R₄ and m has the same meaning as defined above).

The N-substituted N-(4-piperidinyl)amide derivative of the present invention has an alkyl group at 1-position of peperidine, and to a terminal carbon atom of the alkyl group are attached a phenyl group (R⁴) and a carboxyl group (C(=O)R⁵).

In contrast, from the view point of the alkyl group placed at 1-position of the piperidine, to the terminal carbon atom in Fentanyl and the compound of the aforementioned formula (A) is attached a phenyl group only. As for the compound of the formula (B), to the terminal carbon atom is attached a methoxycarbonyl group only. As for the compound of the formula (D), to the terminal carbon atom are attached a phenyl group and an amino acid via a carbonyl group. Therefore, there are distinct difference between these compounds and the compound of the invention.

As for the substituent of the phenyl group which is bonded to the nitrogen atom of the amide attached to the piperidine at 4-position, the compound of the present invention is characterized in that the substituent R² is an amino group, an acetylamino group, an ureido group, or a group of -NR'R" such as a guanidino group.

In contrast, the compound of the aforementioned formula (C) has no substituents on the phenyl group, and the compounds of Reference Examples 1 to 3 described in Patent publication 5 as concrete examples for the compounds of the aforementioned formula (E) have no substituents on the phenyl group. In Patent publication 5, the substituents represented by R₂ in the compounds of the formula (D) are only a halogen atom, an alkyl group having 1 to 6 carbon atoms, an alkoxy group having 1 to 6 carbon atoms, and an alkyl group having 1 to 6 carbon atoms which is substituted with 1 to 3 halogen atoms.

Accordingly, there are structural difference between these compounds and the compound of the invention.

In Non-patent publication 1, there is a description that the narcotic effect of the compound of the formula (C) was not confirmed. In Patent publication 5, the compound of the formula (E) is described merely as an intermediate compound for the preparation of the compound of the formula (D), and no pharmacological effect is described.

In Patent publications 1 and 2, as for the compounds included in the compounds of the formulas (A) and (B), the descriptions for the substituents of the phenyl group bonded to the nitrogen atom of the amide attached to the piperidine at 4-position are limited to a halogen atom, a lower alkyl group, a lower alkoxy group and a trifluoromethyl group.

### [DISCLOSURE OF INVENTION]

It is an object of the invention to provide novel N-substituted N-(4-piperidinyl)amide derivatives and their salts which have an analgesic effect.

The present invention relates to compounds having the following formula (I) or salts thereof: 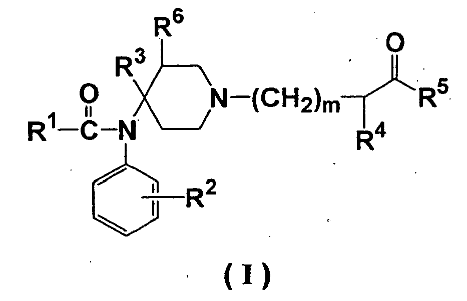 in which
R¹ represents an alkyl group having 1 to 6 carbon atoms, a cycloalkyl group having 3 to 8 ring-forming atoms, an alkyl group having 1 to 6 carbon atoms which has an alkoxy substituent having 1 to 6 carbon atoms, or a heterocyclic group having 5 or 6 ring-forming atoms which optionally has 1 to 3 substituents selected from the group consisting of halogen atoms and alkyl groups having 1 to 6 carbon atoms;
R² represents an amino group, an alkylamino group having 1 to 6 carbon atoms, a dialkylamino group having 2 to 12 carbon atoms, an acylamio group having 1 to 6 carbon atoms, an alkyl(thiocarbonyl)amino group having 2 to 6 carbon atoms, a ureido group, a thioureido group, a guanidino group, an alkoxycarbonylamino group having 2 to 6 carbon atoms, or an alkylsulfonylamino group having 1 to 6 carbon atoms, provided that R² is placed in ortho or meta position;
R³ represents a hydrogen atom, an alkoxycarbonyl group having 2 to 8 carbon atoms, or a methyl group having an alkoxy substituent having 1 to 6 carbon atoms;
R⁴ represents a phenyl or thienyl group which optionally has 1 to 3 substituents selected from the group consisting of a halogen atom, an alkyl group having 1 to 6 carbon atoms, an alkoxy group having 1 to 6 carbon atoms, an alkyl group having 1 to 6 carbon atoms which is substituted with a halogen atom, a nitro group, a cyano group, and an amino group;
R⁵ represents OR⁷ or NR⁸R⁹ in which each of R⁷, R⁸ and
R⁹ is a hydrogen atom, an alkyl group having 1 to 6 carbon atoms, or an aralkyl group having an alkyl moiety of 1 to 6 carbon atoms and an aryl moiety which optionally has a substituent selected from the group consisting of an alkyl group having 1 to 6 carbon atoms and an alkoxy group having 1 to 6 carbon atoms;
R⁶ represents a hydrogen atom or a methyl group;
   and
m represents 1 or 2.

Further, the present invention relates to an analgesic containing a compound of the formula (I) or a salt thereof.

### [PREFERRED EMBODIMENTS OF INVENTION]

The invention is further described below in detail.

In the formula (I), R¹ can be an alkyl group having 1 to 6 carbon atoms, such as methyl, ethyl, propyl, isopropyl, butyl, i-butyl, t-butyl, or pentyl (suitably ethyl); a cycloalkyl group having 3 to 7 ring-forming atoms, such as cyclopropyl, cyclopentyl, or cyclohexyl (suitably cyclopropyl); an alkyl group having 1 to 6 carbon atoms (such as methyl, ethyl, propyl, isopropyl, butyl, i-butyl, t-butyl, or pentyl) which is substituted with an alkoxy group having 1 to 6 carbon atoms (such as methoxy, ethoxy, propyloxy, isopropyloxy, butyloxy, i-butyloxy, t-butyloxy, or pentyloxy), such as ethoxyethyl or methoxymethyl (suitably methoxymethyl); or a heterocyclic group having 5 or 6 ring-forming atoms such as a furyl group, a thiazolyl group or a thienyl group, which optionally has 1 to 3 halogen substituents (such as chloride, bromide, or fluoride) or 1 to 3 alkyl substituents having 1 to 6 carbon atoms (such as methyl, ethyl, propyl, isopropyl, butyl, i-butyl, t-butyl, or pentyl).

R² can be an amino group, an alkylamino group having 1 to 6 carbon atoms, such as methylamino, ethylamino, propylamino, butylamino, or pentylamino; a dialkylamino group having 2 to 12 carbon atoms, such as dimethylamino, diethylamino, or dipropylamino; an acylamino group having 1 to 6 carbon atoms, such as formylamino, acetylamino, or propionylamino; an alkyl(thiocarbonyl) amino group having 2 to 6 carbon atoms such as methyl(thiocarbonyl)amino or ethyl(thiocarbonyl)amino; ureido; thioureido; guanidino; an alkoxycarbonylamino group having 2 to 6 carbon atoms, such as methoxycarbonylamino, ethoxycarbonylamino, propyloxycarbonylamino, butoxycarbonyl, or t-butoxycarbonylamino; or an alkylsulfonylamino group having 1 to 6 carbon atoms, such as methylsulfonylamino, ethylsulfonylamino, or propylsulfonylamino.

R³ can be a hydrogen atom; an alkoxycarbonyl group having 2 to 8 carbon atoms, such as methoxycarbonyl, ethoxycarbonyl, or propyloxycarbonyl (suitably methoxycarbonyl); a methyl group substituted with an alkoxy group having 1 to 6 carbon atoms (such as methoxy, ethoxy, propyloxy, isopropyloxy, butyloxy, i-butyloxy, t-butyloxy, or pentyloxy) such as methoxymethyl.

R⁴ can be a phenyl group or a thienyl group, which optionally has 1 to 3 substituents.

Examples of the substituents include a halogen atom such as fluoride, chloride or bromide; an alkyl group having 1 to 6 carbon atoms, such as methyl, ethyl, propyl, isopropyl, butyl, i-butyl, t-butyl or pentyl; an alkoxy group having 1 to 6 carbon atoms, such as methoxy, ethoxy, propyloxy, isopropyloxy, butyloxy, i-butyloxy, t-butyloxy, or pentyloxy; an alkyl group having 1 to 6 carbon atoms (such as methyl, ethyl, propyl, isopropyl, butyl, i-butyl, t-butyl, or pentyl), which is substituted with 1 to 3 halogen atoms (such as fluoride, bromide, or chloride) such as trifluoromethyl, chloromethyl, 2-chloroethyl, 2-bromoethyl, or 2-fluoroethyl; a nitro group; a cyano group; or an amino group.

Each of R⁷, R⁸ and R⁹ is a hydrogen atom; an alkyl group having 1 to 6 carbon atom, such as methyl, ethyl, propyl, isopropyl, butyl, i-butyl, t-butyl, or pentyl; or an aralkyl group (the alkyl moiety has 1 to 6 carbon atoms; and the aryl moiety can have 1 to 3 substituents which are alkyl groups (e.g., methyl) having 1 to 6 carbon atoms or alkoxy groups (e.g., methoxy) having 1 to 6 carbon atoms. Suitable substituents are hydrogen, methyl, or ethyl.
(1) A suitable compound of the invention is a compound of the formula (I) in which R⁵ represents OR⁷ (R⁷ is a hydrogen atom, an alkyl group having 1 to 6 carbon atoms, or an aralkyl group having an alkyl moiety of 1 to 6 carbon atoms and an aryl moiety which optionally has a substituent selected from the group consisting of an alkyl group having 1 to 6 carbon atoms and an alkoxy group having 1 to 6 carbon atoms) or a salt thereof.
(2) Another suitable compound of the invention is a compound of the formula (I) in which R⁵ is a hydroxyl group, or a salt thereof.
(3) A further suitable compound of the invention is a compound of the formula (I) or a compound of (1) or (2) above, in which R⁶ is a hydrogen atom, or a salt thereof.
(4) A still further suitable compound of the invention is a compound of the formula (I) or a compound of any one of (1) to (3) above, in which m is 1, or a salt thereof.
(5) A still further suitable compound of the invention suitably is a compound of the formula (I) or a compound of any one of (1) to (4) above, in which R² is an amino group, a acetylamino group, a ureido group, or a guanidino group, or a salt thereof.
(6) A still further suitable compound of the invention suitably is a compound of the formula (I) or a compound of any one of (1) to (5) above, in which R² is placed, in the meta-position or a salt thereof.
(7) A still further suitable compound of the invention suitably is a compound of the formula (I) or a compound of any one of (1) to (6) above, in which R³ is a hydrogen atom or an alkoxycarbonyl group having 2 to 8 carbon atoms, or a salt thereof.
(8) A still further suitable compound of the invention is a compound of the formula (I) or a compound of any one of (1) to (6) above, in which R³ is a hydrogen atom, or a salt thereof.
(9) A still further suitable compound of the invention suitably is a compound of the formula (I) or a compound of any one of (1) to (6) above, in which R³ is an alkoxycarbonyl group having 2 to 8 carbon atoms, or a salt thereof.
(10) A still further suitable compound of the invention is a compound of the formula (I) or a compound of any one of (1) to (9) above, in which R¹ is an alkyl group having 1 to 6 carbon atoms or a heterocyclic group having 5 or 6 ring-forming atoms which optionally has 1 to 3 substituents selected from the group consisting of halogen atoms and alkyl groups having 1 to 6 carbon atoms, or a salt thereof.
(11) A still further suitable compound of the invention suitably is a compound of the formula (I) or a compound of any one of (1) to (9) above, in which R¹ is an alkyl group having 1 to 6 carbon atoms, or a salt thereof.
(12) A still further suitable compound of the invention is a compound of the formula (I) or a compound of any one of (1) to (9) above, in which R¹ is a heterocyclic group having 5 or 6 ring-forming atoms which optionally has 1 to 3 substituents selected from the group consisting of halogen atoms and alkyl groups having 1 to 6 carbon atoms, or a salt thereof.
(13) A still further suitable compound of the invention is a compound of the formula (I) or a compound of any one of (1) to (9) above, in which R¹ is a furyl group or a thienyl group, or a salt thereof.
(14) A still further suitable compound of the invention is a compound of the formula (I) or a compound of any one of (1) to (9) above, in which R¹ is a furyl group, or a salt thereof.
(15) A still further suitable compound of the invention is a compound of the formula (I) or a compound of any one of (1) to (14) above, in which R⁴ is a phenyl group, or a salt thereof.
(16) A still further suitable compound of the invention is a compound of the formula (I) or a compound of any one of (1) to (14) above, in which R⁴ is a thienyl group, or a salt thereof.
(17) A still further suitable compound of the invention is selected from the group consisting of:
   3-[4-[N-(3-guanidinophenyl)-N-propionylamino]-piperidin-1-yl]-2-phenylpropionic acid,
   3-[4-[N-(3-aminophenyl)-N-(2-furoyl)amino]-4-methoxycarbonylpiperidin-1-yl]-2-phenylpropionic acid,
   3-[4-[N-(2-furoyl)-N-(3-ureidophenyl)amino]-4-methoxycarbonylpiperidin-1-yl]-2-phenylpropionic acid,
   3-[4-[N-(3-acetylaminophenyl)-N-(2-furoyl)amino]-4-methoxycarbonylpiperidin-1-yl]-2-phenylpropionic acid,
   3-[4-[N-(3-acetylaminophenyl)-N-propionylamino]-4-methoxycarbonylpiperidin-1-yl]-2-phenylpropionic acid,
      and
   cis-3-[4-[N-(2-furoyl)-N-(3-ureidophenyl)amino]-3-methylpiperidin-1-yl]-2-phenylpropionic acid,
      or salts thereof.
(18) An analgesic of the invention which contains an compound of the formula (I) or a salt thereof, or a any one of the compounds of (1) to (17) above as an effective ingredient suitably has a peripheral analgesic effect.

The compounds of the invention having the aforementioned formula (I) can be in the form of a geometrical isomer, a diastereomer, or an optical isomer. These isomers are included within the invention.

The compounds of the invention can be in the form of a pharmaceutically acceptable salts such as salts with an inorganic acid (e.g., hydrochloric acid or sulfuric acid) or an organic acid (e.g., oxalic acid, citric acid, or tartaric acid), a sodium salt, or a potassium salt.

The compounds of the formula (1) according to the invention can be prepared by the below-described processes.

### 1. Compounds of the formula (I) in which R⁵ is represented by OR⁷ (R⁷ is not a hydrogen atom)

The compounds (d) of the formula (I) in which R⁵ is represented by OR⁷ can be prepared by the below-mentioned A-process or B-process.

### [A-process]

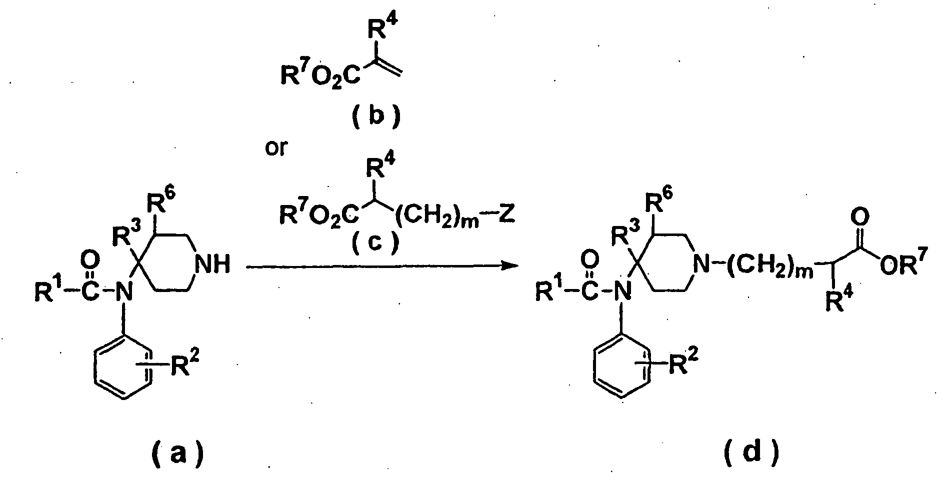

In the scheme, Z is a leaving group such as chloride, bromide, iodide, mesyloxy, tosyloxy, or acetyloxy; and R¹, R², R³, R⁴, R⁶, R⁷, and m have the aforementioned meanings, provided that R⁷ is not hydrogen.
1) The starting material (a) can be prepared by the known methods (P.G.H. Van Daele et al., Arzneum. -Forsch, Drug Res., 1976, 26, 1521; P.L. Feldman, M.F.J. Brackeen, J. Org. Chem., 1990, 55, 4207; N. Lalinde et al., J. Med. Chem., 1990, 33, 2876, etc.) or methods similar to the known methods.
2) The preparation of the compound (d) of the invention from the starting material (a) can be performed in the following manner.
   (1) The starting material (a) and α,β-unsaturated ester (b) are subjected to Michael reaction in an inert solvent such as acetonitrile at a temperature from room temperature to 80°C to give the compound (d) of the invention (in which m is 1).
   (2) The starting material (a) and the compound of the formula (c) are subjected to reaction in an inert solvent such as dichloromethane, acetonitrile, 4-methyl-2-pentanone, or N,N-dimethylformamide in the presence of a base such as sodium carbonate or potassium carbonate, to give the compound (d) of the invention (in which m is 1 or 2). The reaction is conducted at a temperature from room temperature to 100°C. In the case, the compound of the formula (c) has chloride or bromide as Z, sodium iodide or potassium iodide is suitably present in the reaction system.

### [B-process]

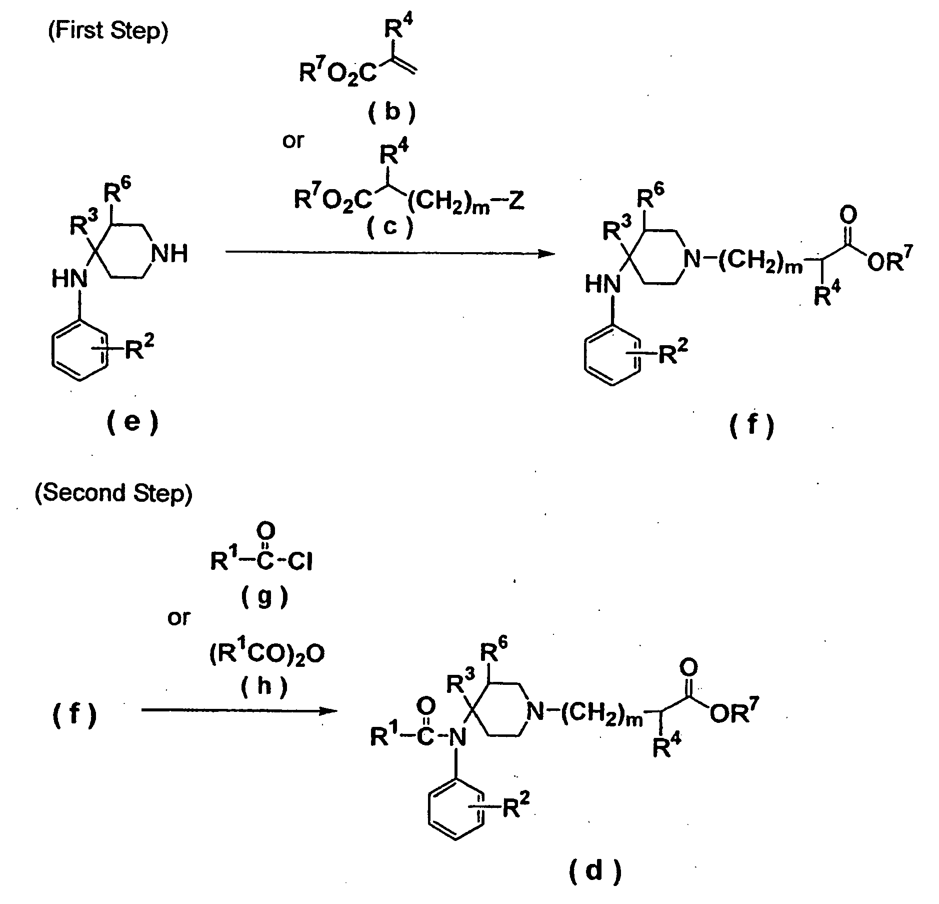

In the scheme, R¹, R², R³, R⁴, R⁶, R⁷, Z, and m have the aforementioned meanings, provided that R⁷ is not hydrogen.
1) The starting material (e) can be prepared by the known methods (P.G.H. Van Daele et al., Arzneum. -Forsch, Drug Res., 1976, 26, 1521; Z-X. Wang et al, J.Med. Chem., 1995, 38, 3652, etc.) or methods similar to the known methods.
2) First step
   The reaction of the starting material (e) with the compound (b) or the compound (c) can be performed in the same manner as described for the aforementioned A-process.
3) Second step
   The acylation of the compound of the formula (f) can be performed by reacting with an acid chloride of the formula (g) in an inert solvent such as dichloromethane, chloroform, or toluene in the presence of a base such as triethylamine or N-methylmorpholine. The reaction temperature is in the range of 0°C to the reflux temperature of the solvent. The acylation of the compound of the formula (f) can be performed by reacting with an acid anhydride at room temperature to 150°C in the presence or absence of an inert solvent such as toluene.

In the case that R² of the compound (d) obtained by the A-process or B-process is an amino group; that is, in the case that the compound (d) is the compound (i), the compound (j) of the invention can be prepared by the below-mentioned C-process.

### [C-process]

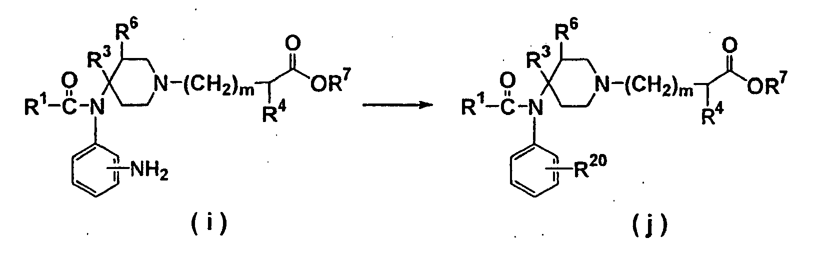

In the scheme, R²⁰ represents acylamino having 1 to 6 carbon atoms, alkyl(thiocarbonyl)amino having 1 to 6 carbon atoms, ureido, thioureido, guanidino, alkoxycarbonylamino having 1 to 6 carbon atoms, or alkylsulfonylamino having 1 to 6 carbon atoms, which is attached to the ortho- or meta-position. R¹, R³, R⁴, R⁶, R⁷, and m have the aforementioned meanings, provided that R⁷ is not hydrogen.

The conversion of the compound of the formula (i) into the compound (j) of the invention can be performed in the below-mentioned manner, depending upon the group of R²⁰.
(1) In the case that R²⁰ is an acylamino group having 1 to 6 carbon atoms
   The compound of the formula (i) is reacted with an acid halide or an acid anhydride.
(2) In the case that R²⁰ is an alkyl(thiocarbonyl)-amino group
   The amino group of the compound of the formula (i) is converted into an acylamino group having 1 to 6 carbon atoms, and the resulting compound is reacted with Lawesson's reagent (Tetrahedron, 1985, 41, 5061, etc.) or the like.
(3) In the case that R²⁰ is ureido
   The compound of the formula (i) is reacted with sodium cyanate or the like in an acidic condition.
(4) In the case that R²⁰ is thioureido
   The compound of the formula (i) is reacted with sodium thiocyanate or the like in an acidic condition.
(5) In the case that R²⁰ is guanidino
   The compound of the formula (i) is reacted with thiourea or guanidine of which amino group is protected by tert-butoxycarbonyl group or the like, and then the protecting group is removed (Tetrahedron Lett., 1993, 34, 7677, J. Org. Chem. 1998, 63, 8432, etc.)
(6) In the case that R²⁰ is an alkoxycarbonylamino group having 1 to 6 carbon atoms
   The compound of the formula (i) is reacted with chlorocarbonate or the like.
(7) In the case that R²⁰ is an alkylsulfonylamino group having 1 to 6 carbon atoms
   The compound of the formula (i) is reacted with sulfonyl halide.

The compound (i), which is a compound of the invention as well as an intermediate compound, can be prepared by the below-mentioned D-process.

### [D-process]

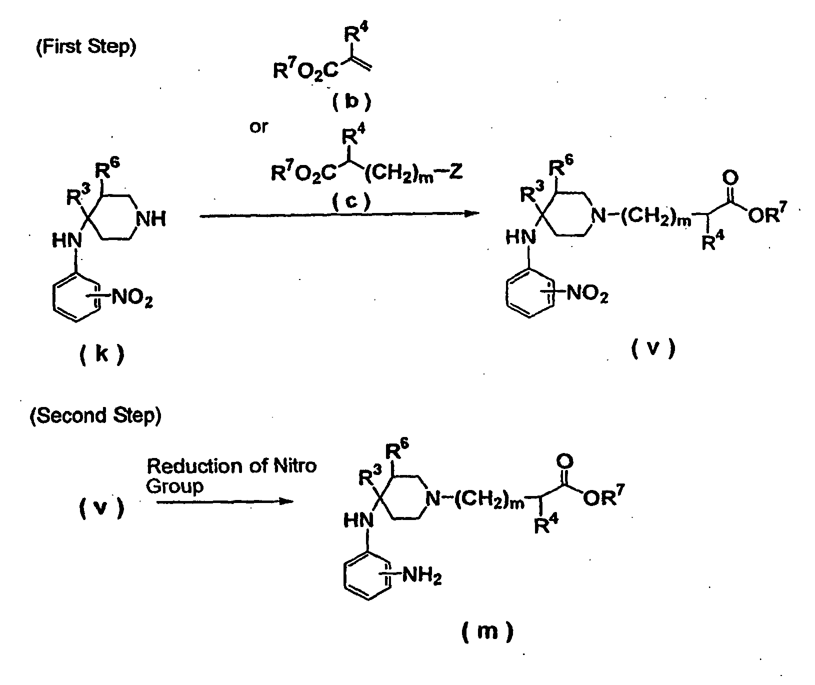

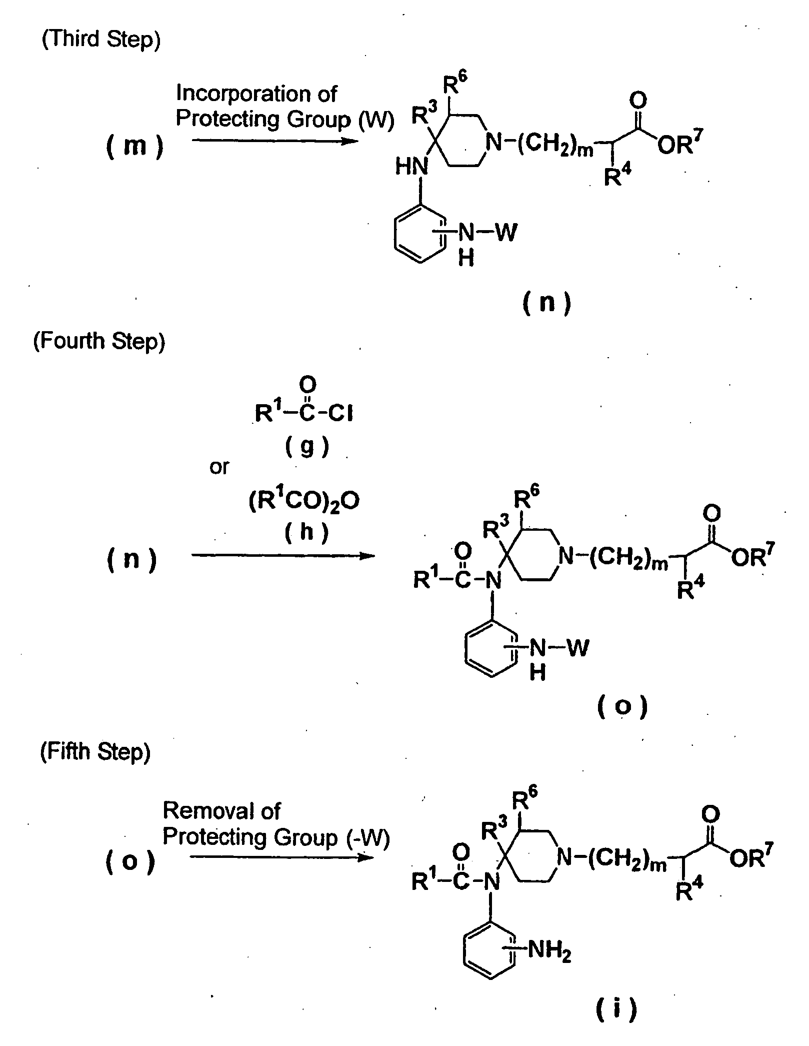

In the scheme, W represents a protecting group such as tert-butoxycarbonyl, benzyloxycarbonyl, or trityl. R¹, R³, R⁴, R⁶, R⁷, Z, and m have the aforementioned meanings, provided that R⁷ is not hydrogen.
1) The starting material (k) can be prepared by the known methods (Z-X. Wang et al., J. Med. Chem., 1995, 38, 3652, etc.) and methods similar to the known methods.
2) First step
   The reaction of the starting material (k) with the compound (b) or compound (c) can be performed in the same manner as described for the aforementioned A-process.
3) Second step
   The conversion of the compound of the formula (v) into the compound of the formula (m) can be performed by reduction in an inert solvent such as methanol, ethanol, water or acetic acid, using a metal or a metal salt (such as zinc, iron, or tin(II) chloride), a sulfur compound (such as sodium hydrosulfite), or catalytic hydrogenation in the presence of a catalyst (e.g., palladium-carbon or platinum (IV) oxide). The reaction temperatures depend on the nature of the reducing agents, but are from room temperature to the refulxing temperature of the solvent.
4) Third step
   The incorporation of a protecting group into the primary amino group of the compound of the formula (m) can be performed in the conventional manner. For instance, in the case that the protecting group is tert-butoxycarbonyl, the protection can be performed by reaction with di-tert-butyl dicarbonate in a solvent (e.g., tetrahydrofuran or water) in the presence of a base (e.g., triethylamine). In the case that the protecting group is trityl, the protection can be performed by reaction with trityl chloride in a solvent (e.g., chloroform) in the presence of a base (e.g., triethylamine).
5) Fourth step
   The acylation of the compound of the formula (n) can be performed in the same manner as described for the second step of the aforementioned B-process.
6) Fifth step
   The removal of the protecting group (W) from the compound of the formula (o) can be performed in the conventional manner. For instance, in the case that the protecting group is tert-butoxycarbonyl or trityl, the compound of the formula (i) can be obtained by acid treatment using trifluoroacetic acid or the like.

The compound (n) which is an intermediate compound for the preparation of the compound of the invention can be prepared by the below-mentioned E-process.

### [E-process]

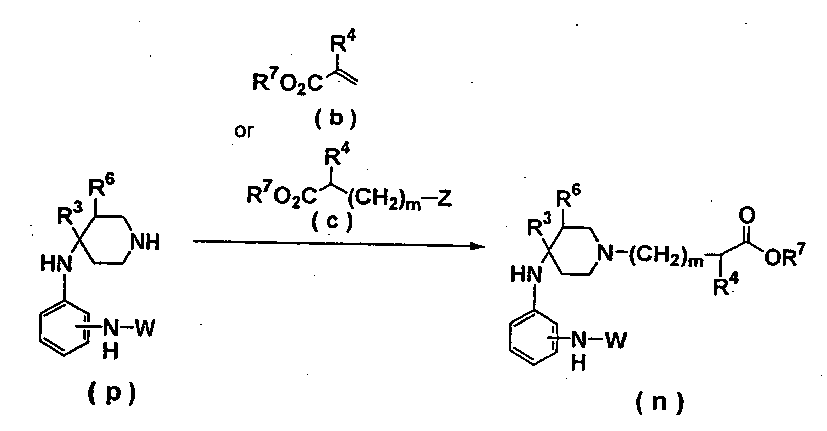

In the scheme, R³, R⁴, R⁶, R⁷, W, Z and m have the aforementioned meanings, provided that R⁷ is not hydrogen.
1) The starting material (p) can be prepared by the known methods (Z-X. Wang et al., J. Med. Chem., 1995, 38, 3652, etc.) or methods similar to the known methods. The compound also can be prepared by reducing the nitro group of the compound (k) illustrated in the D-process into an amino group, and incorporating a protecting group (W).
2) The reaction of the starting material (p) with the compound (b) or compound (c) can be performed in the same manner as described for the A-process.

### 2. Compounds of the formula (I) in which R⁵ is represented by OH

The compounds (p) of the formula (I) in which R⁵ is represented by OH can be prepared by the below-mentioned F-process.

### [F-process]

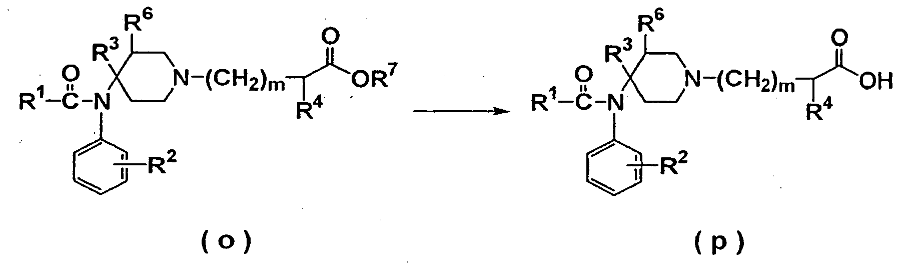

In the scheme, R¹, R², R³, R⁴, R⁶, R⁷, and m have the aforementioned meanings, provided that R⁷ is not hydrogen.
(1) In the case that R⁷ is benzyl
   The compound (p) of the invention can be prepared by catalytic hydrogenation in a solvent (e.g., methanol or ethanol) using a catalyst (e.g., palladium-carbon).
(2) In the case that R⁷ is tert-butyl, p-methoxybenzyl, or 2,4,6-trimethylbenzyl
   The compound (p) of the invention can be prepared by acid-treatment using trifluoroacetic acid or the like.
(3) In the case that R⁷ is an alkyl group having 1 to 6 carbon atoms other than tert-butyl

The compound (p) of the invention can be prepared by treatment with a strong base (e.g., lithium hydroxide, sodium hydroxide, or potassium hydroxide) in a solvent mixture of water and a compatible solvent (e.g., methanol, ethanol, or tetrahydrofuran). The reaction temperature is in the range of 0 to 60°C. Also employable is a conventional acidic hydrolysis using hydrochloric acid or the like. The reaction temperature is in the range of 20 to 120°C.

### 3. Compounds of the formula (I) in which R⁵ is represented by NR⁸R⁹

The compound (r) of the invention having the formula (I) in which R⁵ is represented by NR⁸R⁹ can be prepared by the below-mentioned G-process.

### [G-process]

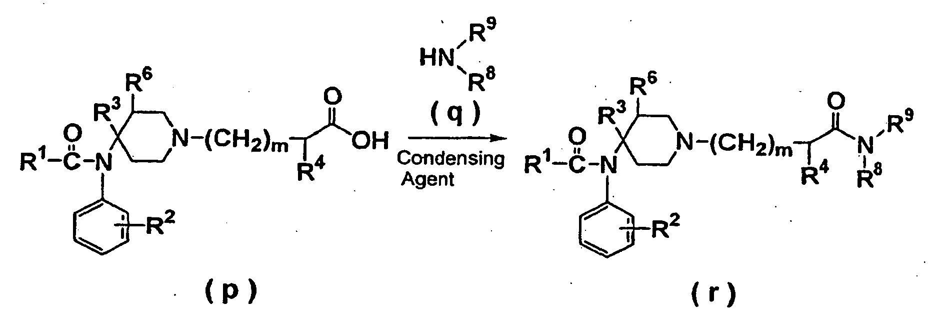

In the scheme, R¹, R², R³, R⁴, R⁶, R⁸, R⁹ and m have the aforementioned meanings.

The condensation of the compound of the formula (p) with the amine (q) can be performed using a condensing agent (e.g., N,N'-dicyclohexylcarbodiimide or 1-(3-dimethylaminopropyl)-3-ethylcarbodiimide hydrochloride) in an inert solvent (e.g., dichlomethane or N,N-dimethylformamide) in the presence or absence of an additive (e.g., 1-hydroxybenzotriazole or N-hydroxysuccinimide). The reaction temperature is in the range of 0 to 60°C. In the case that the condensing agent is 1-(3-dimethylaminopropyl)-3-ethylcarbodiimide hydrochloride or the amine (q) is a salt such as hydrochloride, a base such as triethylamine or N-methylmorpholine is incorporated into the reaction system.

The representative examples of the compounds according to the invention are described below.

### [Representative examples of the compounds of the invention]

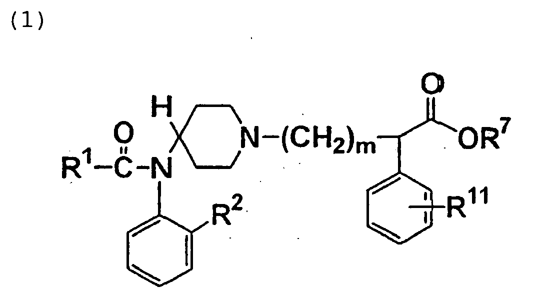

In the formula, R¹, R², R⁷, R¹¹ and m are described in Table 1.

**Table 1**

| R¹ | R² | R⁷ | R¹¹ | m |
|---|---|---|---|---|
| Et | acetylamino | Me | H | 1 |
| Et | acetylamino | H | H | 1 |
| Et | ureido | H | H | 1 |
| cyclopropyl | acetylamino | Et | H | 1 |
| cyclopropyl | amino | H | H | 1 |
| methoxymethyl | dimethylamino | H | H | 1 |
| 2-furyl | acetylamino | H | H | 1 |
| 2-furyl | acetylamino | Bn | H | 2 |
| 2-furyl | ureido | H | 2-F | 1 |
| 3-furyl | acetylamino | H | 3-OMe | 1 |

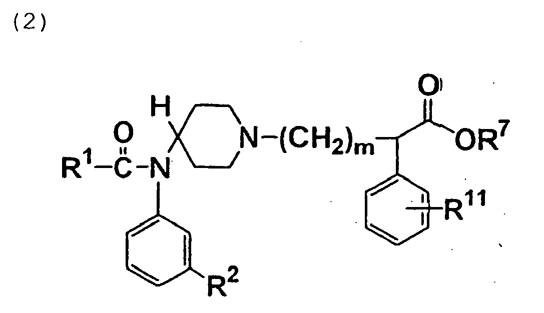

In the formula, R¹, R², R⁷, R¹¹ and m are described in Tables 2 to 4.

**Table 2**

| R¹ | R² | R⁷ | R¹¹ | m |
|---|---|---|---|---|
| Et | amino | Bn | H | 1 |
| Et | acetylamino | Et | H | 1 |
| Et | acetylamino | H | H | 1 |
| Et | propionylamino | H | 4-F | 2 |
| Et | methoxycarbonylamino | Me | 4-NH₂ | 1 |
| Et | t-butoxycarbonylamino | Bn | H | 1 |
| Et | ureido | Bn | H | 1 |
| Et | ureido | H | H | 1 |
| Et | guanidino | Bn | H | 1 |
| Et | guanidino | H | H | 1 |

**Table 3**

| R¹ | R² | R⁷ | R¹¹ | m |
|---|---|---|---|---|
| cyclopropyl | acetylamino | t-Bu | H | 1 |
| cyclopropyl | guanidino | H | H | 1 |
| methoxymethyl | acetylamino | Me | 3,4-Cl | 1 |
| methoxymethyl | acetylamino | Bu | H | 1 |
| 2-furyl | amino | Bn | H | 1 |
| 2-furyl | methylamino | H | H | 1 |
| 2-furyl | dimethylamino | H | 3-CF3 | 1 |
| 2-furyl | acetylamino | Et | H | 1 |
| 2-furyl | acetylamino | H | H | 1 |

**Table 4**

| R¹ | R² | R⁷ | R¹¹ | m |
|---|---|---|---|---|
| 2-furyl | t-butoxycarbonylamino | Bn | H | 1 |
| 2-furyl | ureido | Bn | H | 1 |
| 2-furyl | ureido | H | H | 1 |
| 2-furyl | guanidino | Bn | H | 1 |
| 2-furyl | guanidino | H | H | 1 |
| 3-furyl | ureido | H | H | 1 |
| 2-thienyl | acetylamino | Me | 4-NO₂ | 1 |
| 2-thienyl | thioureido | H | H | 1 |
| 3-thienyl | acetylamino | Et | H | 1 |
| 5-isoxazolyl | dimethylamino | Et | H | 1 |

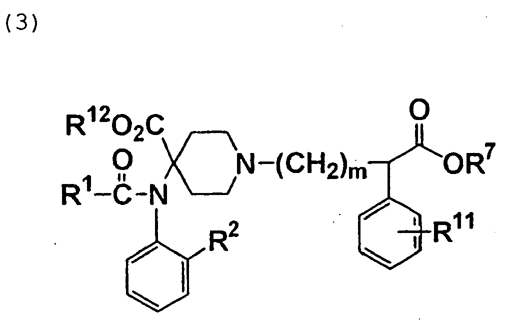

In the formula, R¹, R², R⁷, R¹¹, R¹² and m are described in Table 5.

**Table 5**

| R¹ | R² | R⁷ | R¹¹ | R¹² | m |
|---|---|---|---|---|---|
| Et | acetylamino | H | H | Me | 1 |
| Et | acetylamino | Me | H | Me | 2 |
| Et | dimethylamino | Me | 2-F | Me | 1 |
| cyclopropyl | acetylamino | Et | H | Et | 1 |
| cyclopropyl | ureido | H | H | Me | 1 |
| methoxymethyl | methylamino | t-Bu | 3-CN | Me | 1 |
| 2-furyl | acetylamino | Me | H | Me | 1 |
| 2-furyl | acetylamino | H | H | Me | 1 |
| 2-furyl | ureido | H | H | Me | 1 |
| 3-furyl | thioureido | Pr | H | Me | 1 |
| 2-thienyl | ureido | Bn | H | Me | 1 |

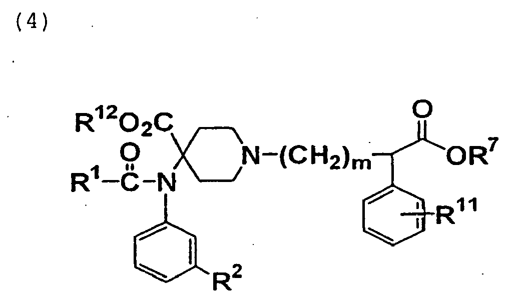

In the formula, R¹, R², R⁷, R¹¹, R¹² and m are described in Tables 6 to 10.

**Table 6**

| R¹ | R² | R⁷ | R¹¹ | R¹² | m |
|---|---|---|---|---|---|
| Et | acetylamino | Bn | H | Me | 1 |
| Et | acetylamino | H | H | Me | 1 |
| Et | acetylamino | Me | H | Me | 2 |
| Et | amino | Pr | 2-F | Me | 1 |
| Et | amino | Bn | H | Me | 1 |
| Et | amino | H | H | Me | 1 |
| Et | ethylamino | H | H | Et | 1 |
| Et | diethylamino | t-Bu | 3-CF₃ | Me | 1 |

**Table 7**

| R¹ | R² | R⁷ | R¹¹ | R¹² | m |
|---|---|---|---|---|---|
| Et | ureido | Bn | H | Me | 1 |
| Et | ureido | H | H | Me | 1 |
| Et | thioureido | H | H | Me | 1 |
| Et | guanidino | Bn | H | Me | 1 |
| Et | guanidino | H | H | Me | 1 |
| cyclopropyl | acetylamino | t-Bu | H | Me | 1 |
| cyclopropyl | acetylamino | H | H | Me | 1 |
| cyclopropyl | thioureido | H | H | Pr | 1 |
| cyclopropyl | guanidino | H | H | Me | 1 |

**Table 8**

| R¹ | R² | R⁷ | R¹¹ | R¹² | m |
|---|---|---|---|---|---|
| methoxymethyl | acetylamino | Me | 4-Me | Me | 1 |
| methoxymethyl | ureido | H | H | Me | 1 |
| 2-furyl | acetylamino | Me | H | Me | 1 |
| 2-furyl | acetylamino | Bn | H | Me | 1 |
| 2-furyl | acetylamino | H | H | Me | 1 |
| 2-furyl | propionylamino | Et | H | Me | 1 |
| 2-furyl | methyl(thio- | H | H | Me | 1 |
| | carbonyl) amino | | | | |
| 2-furyl | methylsulfonylamino | t-Bu | H | Me | 1 |

**Table 9**

| R¹ | R² | R⁷ | R¹¹ | R¹² | m |
|---|---|---|---|---|---|
| 2-furyl | methoxycarbonylamino | Et | 3-OMe | Me | 1 |
| 2-furyl | t-butoxycarbonylamino | Bn | H | Me | 1 |
| 2-furyl | amino | Et | H | Me | 1 |
| 2-furyl | amino | Bn | H | Me | 1 |
| 2-furyl | amino | H | H | Me | 1 |
| 2-furyl | methylamino H | H | H | Me | 1 |
| 2-furyl | dimethylamino | Et | H | Me | 1 |
| 2-furyl | ureido | Bn | H | Me | 1 |
| 2-furyl | ureido | H | H | Me | 1 |

**Table 10**

| R¹ | R² | R⁷ | R¹¹ | R¹² | m |
|---|---|---|---|---|---|
| 2-furyl | thioureido | H | H | Me | 1 |
| 2-furyl | guanidino | Bn | H | Me | 1 |
| 2-furyl | guanidino | H | H | Me | 1 |
| 3-furyl | dimethylamino | Et | H | Me | 1 |
| 2-thienyl | acetylamino | Me | H | Me | 1 |
| 2-thienyl | thioureido | H | H | Me | 1 |
| 3-thienyl | acetylamino | Et | 4-CN | Me | 1 |
| 5-isoxazolyl | dimethylamino | Et | H | Me | 1 |

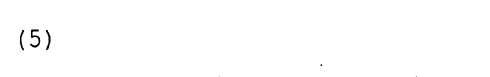

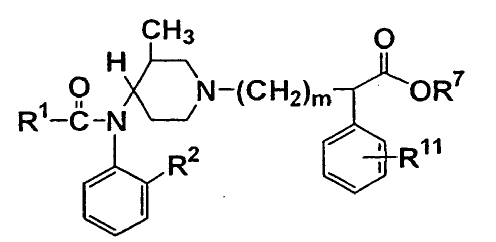

In the formula, R¹, R², R⁷, R¹¹ and m are described in Table 11.

**Table 11**

| R¹ | R² | R⁷ | R¹¹ | m |
|---|---|---|---|---|
| Et | acetylamino | Me | H | 1 |
| Et | acetylamino | H | H | 1 |
| Et | ureido | H | H | 1 |
| cyclopropyl | amino | H | H | 1 |
| methoxymethyl | thioureido | H | H | 1 |
| 2-furyl | dimethylamino | Bn | H | 1 |
| 2-furyl | ureido | H | 2-F | 1 |
| 2-thienyl | ureido | Et | H | 1 |

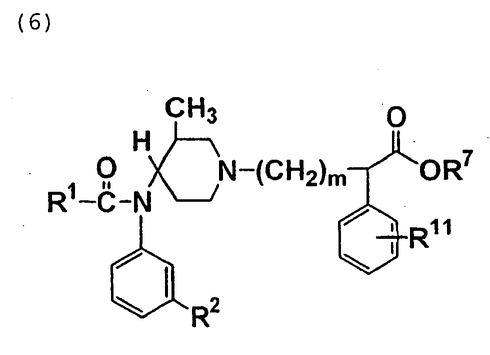

In the formula, R¹, R², R⁷, R¹¹ and m are described in Tables 12 and 13.

**Table 12**

| R¹ | R² | R⁷ | R¹¹ | m |
|---|---|---|---|---|
| Et | acetylamino | H | H | 1 |
| Et | dimethylamino | t-Bu | 3-F | 2 |
| Et | ureido | H | H | 1 |
| Et | guanidino | H | H | 1 |
| cyclopropyl | thioureido | H | H | 1 |
| methoxymethyl | acetylamino | Me | 3,4-Cl | 1 |
| 2-furyl | acetylamino | H | H | 1 |
| 2-furyl | methyl(thiocarbonyl)amino | H | H | 1 |

**Table 13**

| R¹ | R² | R⁷ | R¹¹ | m |
|---|---|---|---|---|
| 2-furyl | t-butoxycarbonylamino | Bn | H | 1 |
| 2-furyl | amino | Bn | H | 1 |
| 2-furyl | amino | H | H | 1 |
| 2-furyl | ureido | Bn | H | 1 |
| 2-furyl | ureido | H | H | 1 |
| 2-furyl | thioureido | H | H | 1 |
| 3-furyl | dimethylamino | Et | H | 1 |
| 2-thienyl | thioureido | H | H | 1 |

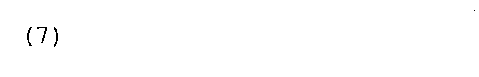

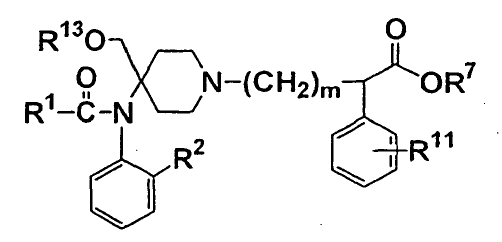

In the formula, R¹, R², R⁷, R¹¹, R¹³ and m are described in Table 14.

**Table 14**

| R¹ | R² | R⁷ | R¹¹ | R¹³ | m |
|---|---|---|---|---|---|
| Et | amino | Me | H | Me | 1 |
| Et | propylamino | H | H | Et | 1 |
| Et | ureido | H | H | Me | 1 |
| cyclopropyl | acetylamino | Et | 4-NH₂ | Me | 1 |
| methoxymethyl | acetylamino | H | H | Me | 1 |
| methoxymethyl | ureido | H | H | Me | 1 |
| 2-furyl | acetylamino | H | H | Me | 1 |
| 2-furyl | ureido | H | 3-Br | Me | 1 |
| 3-furyl | acetylamino | t-Bu | 4-OMe | Me | 1 |
| 2-thienyl | ureido | Bu | H | Me | 1 |

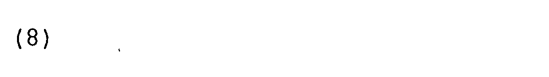

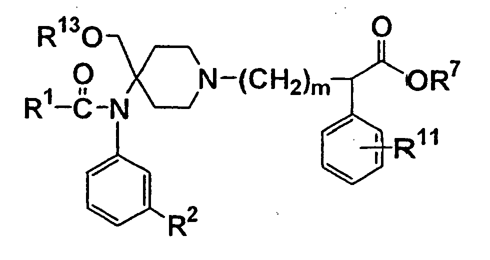

In the formula, R¹, R², R⁷, R¹¹, R¹³ and m are described in Tables 15 and 16.

**Table 15**

| R¹ | R² | R⁷ | R¹¹ | R¹³ | m |
|---|---|---|---|---|---|
| Et | acetylamino | H | H | Me | 1 |
| Et | dimethylamino | t-Bu | H | Me | 2 |
| Et | ureido | Bn | H | Me | 1 |
| Et | ureido | H | H | Me | 1 |
| Et | guanidino | H | H | Me | 1 |
| cyclopropyl | thioureido | H | H | Pr | 1 |
| cyclopropyl | guanidino | H | 2-F | Me | 1 |

**Table 16**

| R¹ | R² | R⁷ | R¹¹ | R ¹³ | m |
|---|---|---|---|---|---|
| methoxymethyl | acetylamino | Me | H | Me | 1 |
| 2-furyl | methyl(thiocarbonyl)amino | H | H | Me | 1 |
| 2-furyl | amino | H | H | Et | 1 |
| 2-furyl | ureido | Bn | H | Me | 1 |
| 2-furyl | thioureido | H | 2,9-F | Me | 1 |
| 2-furyl | guanidino | H | H | Me | 1 |
| 2-thienyl | thioureido | H | H | Me | 1 |

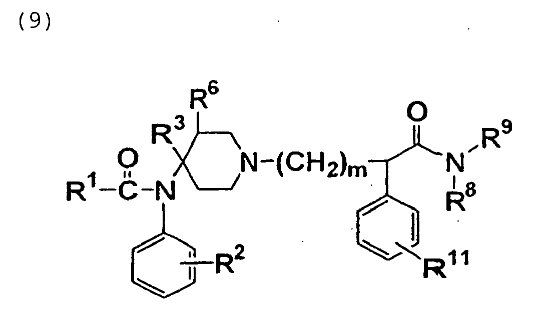

In the formula, R¹, R², R³, R⁸, R⁹, R¹¹ and m are described in Tables 17 and 18.

**Table 17**

| R¹ | R² | R³ | R⁶ | R⁸ | R⁹ | R¹¹ | m |
|---|---|---|---|---|---|---|---|
| Et | o-acetylamino | CO₂Me | H | Me | Me | H | 1 |
| Et | m-acetylamino | CO₂Me | H | H | H | 4-F | 1 |
| Et | m-acetylamino | CO₂Me | Me | CH₂CH₂Ph | H | H | 1 |
| Et | o-ureido | H | H | Me | Me | H | 1 |
| Et | m-ureido | H | H | Et | H | H | 1 |
| Et | m-guanidino | CH₂OMe | H | Et | Et | H | 1 |
| cyclopropyl | m-acetylamino | CO₂Me | H | Et | Et | H | 2 |
| cyclopropyl | m-ureido | H | Me | Bu | H | 3-CN | 1 |
| cyclopropyl | m-guanidino | H | H | Me | Me | H | 1 |
| methoxymethyl | m-ethylamino | CO₂Me | H | Bn | Me | H | 1 |

**Table 18**

| R¹ | R² | R³ | R⁶ | R⁸ | R⁹ | R¹¹ | m |
|---|---|---|---|---|---|---|---|
| methoxymethyl 1 | o-dimethylamino | CO₂Me | H | Me | Me | 2-OMe | |
| methoxymethyl | m-thioureido | CH₂OMe | H | Et | Et | H | 1 |
| 2-furyl | o-acetylamino | H | Me | Et | Et | H | 1 |
| 2-furyl | o-amino | CO₂Me | H | Bn | Me | H | 1 |
| 2-furyl | m-methylamino | CH₂OMe | H | Et | Et | H | 1 |
| 2-furyl | m-ureido | CO₂Me | Me | Et | Et | H | 1 |
| 2-furyl | m-guanidino | H | H | H | H | H | 1 |
| 3-furyl | m-acetylamino | CO₂Me | H | i-Pr | H | H | 1 |
| 2-thienyl | o-acetylamino | CO₂Me | H | Bu | Bu | H | 1 |
| 2-thienyl | o-dimethylamino | CH₂OMe | H | Bn | H | H | 1 |

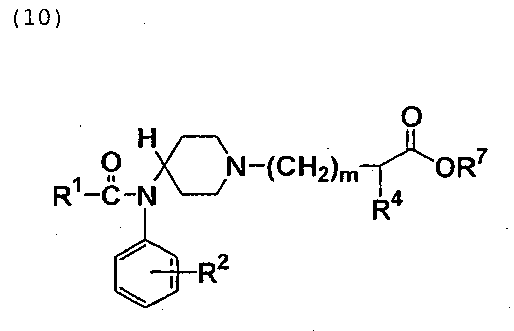

In the formula, R¹, R², R⁴, R⁷ and m are described in Table 19.

**Table 19**

| R¹ | R² | R⁴ | R⁷ | m |
|---|---|---|---|---|
| Et | o-acetylamino | 2-thienyl | H | 1 |
| Et | m-ureido | 2-thienyl | H | 1 |
| cyclopropyl | m-acetylamino | 3-thienyl | H | 1 |
| cyclopropyl | o-amino | 2-thienyl | H | 1 |
| methoxymethyl | m-dimethylamino | 2-thienyl | H | 1 |
| 2-furyl | o-acetylamino | 2-thienyl | Bn | 1 |
| 2-furyl | o-acetylamino | 2-thienyl | H | 1 |
| 2-furyl | m-ureido | 3-thienyl | H | 1 |
| 2-furyl | m-guanidino | 2-thienyl | H | 1 |
| 3-furyl | m-acetylamino | 2-thienyl | H | 1 |

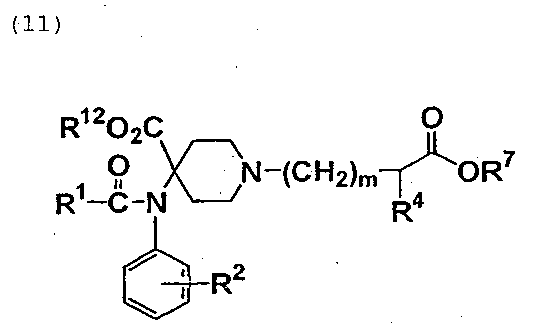

In the formula, R¹, R², R⁴, R⁷, R¹² and m are described in Tables 20 and 21.

**Table 20**

| R¹ | R² | R⁴ | R⁷ | R¹² | m |
|---|---|---|---|---|---|
| Et | m-amino | 2-thienyl | H | Me | 1 |
| Et | o-dimethylamino | 3-thienyl | H | Me | 1 |
| Et | o-acetylamino | 3-thienyl | H | Me | 1 |
| Et | m-acetylamino | 2-thienyl | AA* | Me | 1 |
| Et | m-acetylamino | 2-thienyl | H | Me | 1 |
| Et | m-ureido | 2-thienyl | H | Me | 1 |
| cyclopropyl | o-acetylamino | 2-thienyl | H | Me | 1 |
| cyclopropyl | m-acetylamino | 2-thienyl | H | Me | 1 |
| cyclopropyl | m-ureido | 3-thienyl | H | Me | 1 |
| methoxymethyl | m-methylamino | 3-thienyl | H | Me | 1 |

| | | | | | |
|---|---|---|---|---|---|
| Remarks: AA*=2,4,6-trimethylbenzyl | | | | | |

**Table 21**

| R¹ | R² | R⁴ | R⁷ | R¹² | m |
|---|---|---|---|---|---|
| 2-furyl | m-amino | 2-thienyl | BB* | Me | 1 |
| 2-furyl | m-amino | 2-thienyl | H | Me | 1 |
| 2-furyl | o-dimethylamino | 3-thienyl | H | Me | 1 |
| 2-furyl | m-acetylamino | 2-thienyl | BB* | Me | 1 |
| 2-furyl | m-acetylamino | 2-thienyl | H | Me | 1 |
| 2-furyl | m-ureido | 2-thienyl | H | Me | 1 |
| 2-furyl | m-thioureido | 2-thienyl | H | Me | 1 |
| 3-furyl | m-guanidino | 2-thienyl | H | Me | 1 |

| | | | | | |
|---|---|---|---|---|---|
| Remarks: BB*=4-methoxybenzyl | | | | | |

Pharmacological tests for the invention are described below.

Pharmacological tests concerning the affinity to µ-receptor by binding assay using [³H] DAMGO and analgesic effect by AcOH writhing tests of the invention compounds were performed. As a result, it was confirmed that the invention compounds have excellent affinity to µ-receptor and an excellent analgesic effect, as described in Example 39 mentioned hereinafter. See Tables 22 and 23.

Further, antagonism tests on the analgesic effect by the acetic acid-writhing test using peripheral and central µ-opioid receptor antagonist indicated that the invention compounds described in the below-mentioned Examples 12, 15, 17, 19 and 32 show a peripheral analgesic effect. See Table 24.

In view of the excellent analgesic effect, the invention compounds represented by the aforementioned formula (1) are of value as analgesics.

Particularly, the invention compounds which selectively act on peripheral µ-receptor are expected to be analgesics which show no adverse effects (drug dependence) caused by the central action.

The invention compound can be administered by oral or non-oral in clinical use.

The invention compounds can be prepared in the form of known pharmaceutical preparations such as tablets, capsules, granules, injections, suppositories, or percutaneous agents.

The dosage of the invention compound for adult generally is approximately 0.01 to 1000 mg/day when it is orally administered, and approximately 0.001 to 100 mg/day when it is administered in the form of an injection. The dosage can be adjusted depending on age and clinical conditions and so on.

The present invention is further described below by the following non-limiting examples and reference examples.

### [Reference Example 1]

Benzyl 3-[4-[3-(tert-butoxycarbonylamino)phenylamino]piperidin-1-yl]-2-phenylpropionate

### (1) 4-(3-Acetylaminophenyl)amino-1-benzylpiperidine

To a solution of 1-benzyl-4-piperidone (1.00 g, 5.28 mmol) and 3'-aminoacetoanilide (913 mg, 6.08 mmol) in toluene (10 mL) was added p-toluenesulfonic acid monohydrate (10 mg). The resulting mixture was heated under reflux overnight using a Dean-Stark trap. The reaction mixture was concentrated under reduced pressure, and the resulting residue was dissolved in methanol (10 mL). After addition of sodium borohydride (230 mg, 6.08 mmol), the mixture was stirred for 2 hours at room temperature. The reaction mixture was poured into an aqueous sodium hydrogencarbonate and extracted with chloroform. The extract was dried over anhydrous sodium sulfate and the solvent was removed under reduced pressure. The residue was purified by silica gel column chromatography (ethyl acetate/methanol=96/4) to give 1.39 g (yield 82%) of the titled compound.

¹H-NMR (CDCl₃, 400 MHz) δ: 1.4-1.6 (2H, m), 1.9-2.2 (4H, m), 2.17 (3H, s), 2.7-2.9 (2H, m), 3.2-3.4 (1H, m), 3.4-3.8 (1H, br), 3.52 (2H, s), 6.33 (1H, d, J=8Hz), 6.54 (1H, d, J=8Hz), 7.0-7.4 (8H, m).

### (2) 4-(3-Aminophenyl)amino-1-benzylpiperidine

Potassium hydroxide (504 mg, 7.64 mmol) was added to a solution of the 4-(3-acetylaminophenyl)amino-1-benzylpiperidine (412 mg, 1.27 mmol) in ethanol (5 mL). The mixture was heated under reflux for 42 hours. The reaction mixture was poured into water and extracted with chloroform. The extract was washed with a saturated brine and dried over anhydrous sodium sulfate. The solvent was removed under reduced pressure to give 457 mg of the titled compound.

¹H-NMR (CDCl₃, 400 MHz) δ: 1.4-1.6 (2H, m), 1.9-2.2 (4H, m), 2.7-2.9 (2H, m), 3.2-3.4 (1H, m), 3.42 (1H, br d, J=8Hz), 3.51(4H, s), 5.94 (1H, t, J=2Hz), 6.03 (1H, dd, J=2, 8Hz), 6.04 (1H, dd, J=2, 8Hz), 6.93 (1H, t, J=8Hz), 7.2-7.4 (5H, m).

### (3) 1-Benzyl-4-[3-(tert-butoxycarbonylamino)phenylamino]piperidine

Triethylamine (0.65 mL) and di-tert-butyl dicarbonate (531 mg, 2.43 mmol) were added to a solution of the 4-(3-aminophenyl)amino-1-benzylpiperidine (456 mg) in a mixture of tetrahydrofuran (3 mL) and water (3 mL). The mixture was stirred overnight at room temperature. The reaction mixture was poured into an aqueous sodium hydrogencarbonate and extracted with chloroform. The extract was dried over anhydrous sodium sulfate and the solvent was removed under reduced pressure. The residue was purified by silica gel column chromatography (chloroform/methanol=98/2) to give 389 mg (yield 80%, two steps) of the titled compound. '

¹H-NMR (CDCl₃, 400 MHz) δ: 1.4-1.6 (2H, m), 1.50 (9H, s), 1.9-2.1 (2H, m), 2.1-2.2 (2H, m), 2.7-2.9 (2H, m), 3.2-3.4 (1H, m), 3.4-3.7 (1H, br.), 3.52 (2H, s), 6.26 (1H, dd, J=2, 8Hz), 6.35 (1H, br s), 6.48 (1H, dd, J=2, 8Hz), 6.82 (1H, br s), 7.03 (1H, t, J=8Hz), 7.2-7.4 (5H, m).

### (4) 4-[3-(tert-Butoxycarbonylamino)phenylamino]-piperidine

To a solution of the 1-benzyl-4-[3-(tert-butoxycarbonylamino)phenylamino]piperidine (385 mg, 1.01 mmol) in methanol (7 mL) was added 10% palladium-carbon (77 mg). The mixture was subjected to catalytic hydrogenation for 5 hours under heating with reflux. The catalyst was filtered off, and the filtrate was concentrated under reduced pressure to give 326 mg of the titled compound.

¹H-NMR (CDCl₃, 400 MHz) δ: 1.2-1.4 (2H, m), 1.51 (9H, s), 2.05 (2H, br d, J=7Hz), 2.6-2.8 (2H, m), 3.0-3.2 (2H, m), 3.3-3.5 (1H, m), 3.4-3.7 (1H, br), 6.27 (1H, dd, J=2, 8Hz), 6.50 (1H, dd, J=2, 8Hz), 6.52 (1H, br s), 6.84 (1H, br s), 7.04 (1H, t, J=8Hz).

### (5) Benzyl 3-[4-[3-(tert-butoxycarbonylamino)phenylamino]piperidin-1-yl]-2-phenylpropionate

To a solution of the 4-[3-(tert-butoxycarbonylamino)phenylamino]piperidine (323 mg) in acetonitrile (8 mL) was added benzyl 2-phenylpropenate (291 mg, 1.22 mmol). The mixture was stirred overnight at room temperature. The reaction mixture was concentrated under reduced pressure. The residue was purified by silica gel column chromatography (n-hexane/ethyl acetate=2/1) to give 451 mg (yield 85%, two steps) of the titled compound.

¹H-NMR (CDCl₃, 400 MHz) δ: 1.2-1.4 (2H, m), 1.51 (9H, s), 1.8-2.0 (2H, m), 2.12 (1H, dt, J=2, 11Hz), 2.29 (1H, dt, J=2, 11Hz), 2.55 (1H, dd, J=5, 13Hz), 2.7-2.8 (1H, m), 2.9-3.0 (1H, m), 3.22 (1H, dd, J=11, 13Hz), 3.1-3.3 (1H, m), 3.49 (1H, br s), 3.89 (1H, dd, J=5, 11Hz), 5.07 (1H, d, J=13Hz), 5.21 (1H, d, J=13Hz), 6.25 (1H, dd, J=2, 8Hz), 6.36 (1H, br s), 6.48 (1H, dd, J=2, 8Hz), 6.81 (1H, br s), 7.03 (1H, t, J=8Hz), 7.2-7.4 (10H, m).

### [Reference Example 2]

### Benzyl 3-[4-[3-(tert-butoxycarbonylamino)phenylamino]-4-methoxycarbonylpiperidin-1-yl]-2-phenylpropionate

### (1) 1-Benzyl-4-(3-nitrophenyl)aminopiperidine-4-carbonitile

To a solution of 3-nitroaniline (1.28 g, 9.27 mmol) in acetic acid (8 mL) was added 1-benzyl-4-piperidone (1.59 mL, 8.58 mmol). The mixture was stirred for one hour at room temperature. To the solution kept at a temperature of lower than 35°C (internal temperature) was added dropwise trimethylsilyl cyanide (1.16 mL, 8.70 mmol). The mixture was stirred overnight at room temperature. The reaction mixture was neutralized with an aqueous potassium carbonate. After addition of diethyl ether, the mixture was stirred overnight. The precipitated yellow crystals were collected by filtration, washed with water and n-hexane/ethyl acetate (3/1), and dried overnight at 50°C under reduced pressure to give 2.22 g (yield 77%) of the titled compound as a yellow crystal.

¹H-NMR (CDCl₃, 400 MHz) δ: 1.9-2.0 (2H, m), 2.40 (2H, dt, J=2, 13Hz), 2.4-2.6 (2H, m), 2.8-2.9 (2H, m), 3.57 (2H, s), 4.09 (1H, s), 7.19 (1H, dd, J=2, 8Hz), 7.2-7.4 (5H, m), 7.38 (1H, t, J=8Hz), 7.6-7.7 (2H, m).

### (2) 1-Benzyl-4-(3-nitrophenyl)aminopiperidine-4-carboxamide

To 97% sulfuric acid (5.4 mL) was slowly added 1-benzyl-4-(3-nitrophenyl)aminopiperidine-4-carbonitrile (2.22 g, 6.60 mmol) under ice-cooling. The mixture was stirred overnight at room temperature. After addition of ice-water, the reaction mixture was neutralized with potassium carbonate. The precipitated product was collected by filtration, washed with warm water to remove inorganic salts, and dried by air at room temperature to give 1.95 g (yield 83%) of the titled compound as a yellow crystal.

¹H-NMR (CDCl₃, 400 MHz) δ: 1.92 (2H, d, J=13Hz), 2.1-2.2 (2H, m), 2.3-2.4 (2H, m), 2.7-2.8 (2H, m), 3.50 (2H, s), 4.36 (1H, s), 5.47 (1H, s), 6.65 (1H, s), 6.89 (1H, dd, J=2, 8Hz), 7.2-7.4 (6H, m), 7.4-7.5 (1H, m), 7.62 (1H, dd, J=2, 8Hz).

### (3) 1-Benzyl-4-(3-nitrophenyl)aminopiperidine-4-carboxamide

To the 1-benzyl-4-(3-nitrophenyl)aminopiperidine-4-carboxamide (1.79 g, 5.06 mmol) were added conc. hydrochloric acid (10 mL) and water (4 mL). The mixture was heated under reflux for 2.5 hours. Then, 6N aqueous sodium hydroxide (11 mL) was added dropwise to the reaction mixture with ice-cooling under the condition that the internal temperature was kept below 40°C. The reaction mixture was cooled to room temperature. The precipitated product was collected by filtration, washed well with water, and dried overnight under reduced pressure at 60°C to give 1.39 g (yield 71%) of the titled compound as a yellow crystal.

¹H-NMR (CD₃OD, 400 MHz) δ: 2.0-3.8 (4H, m), 3.2-3.6 (4H, m), 4.37 (2H, s), 7.01 (1H, d, J=8Hz), 7.34 (1H, t, J=8Hz), 7.5-7.6 (7H, m).

### (4) Methyl 1-benzyl-4-(3-nitrophenyl)aminopiperidine-4-carboxylate

Thionyl chloride (0.41 mL, 5.6 mmol) was added dropwise over 0.5 hour to methanol (4 mL) under ice-cooling. To the resulting solution was added 1-benzyl-4-(3-nitrophenyl)aminopiperidine-4-carboxylic acid (517 mg, 1.45 mmol). The mixture was heated under reflux for 5.5 hours, and then stirred overnight at room temperature. After addition of an aqueous potassium carbonate, the reaction mixture was extracted with chloroform. The extract was washed with water, dried over anhydrous sodium sulfate, and the solvent was removed under reduced pressure to give 532 mg (yield 99%) of the titled compound as a brown oil.

¹H-NMR (CDCl₃, 400 MHz) δ: 2.0-2.1 (2H, m), 2.2-2.4 (4H, m), 2.6-2.7 (2H, m), 3.52 (2H, s), 3.72 (3H, s), 4.19 (1H, s), 6.81 (1H, dd, J=2, 8Hz), 7.2-7.3 (6H, m), 7.40 (1H, t, J=2Hz), 7.56 (1H, dd, J=2, 8Hz).

### (5) Methyl 4-(3-nitrophenyl)aminopiperidine-4-carboxylate hydrochloride

To a solution of the methyl 1-benzyl-4-(3-nitrophenyl)aminopiperidine-4-carboxylate (548 mg, 1.48 mmol) and diisopropylethylamine (0.13 mL, 0.74 mmol) in dichloroethane (4 mL) was added dropwise a solution of 1-chloroethyl chloroformate (233 mg, 1.63 mmol) in dichloroethane (2 mL) under ice-water cooling over 20 minutes, and stirred for 1.5 hours at room temperature, after evaporation of the solvent under reduced pressure. The residue was dissolved in methanol (4 mL) and stirred at 45°C for 45 minutes. The precipitated crystal was collected by filtration and washed with ethyl acetate to give 371 mg (yield 79%) of the titled compound as a yellow crystal.

¹H-NMR (CD₃OD, 400 MHz) δ: 2.3-2.4 (4H, m), 3.2-3.4 (4H, m), 3.70 (3H, s), 6.98 (1H, dd, J=2, 8Hz), 7.34 (1H, t, J=8Hz), 7.44 (1H, t, J=2Hz), 7.53 (1H, dd, J=2, 8Hz).

### (6) Benzyl 3-[4-methoxycarbonyl-4-(3-nitrophenyl)amino-piperidin-1-yl]-2-phenylpropionate

To a solution of the methyl 4-(3-nitrophenyl)amino-piperidine-4-carboxylate hydrochloride (221 mg, 0.70 mmol) in acetonitrile(3 mL)-methanol(0.5 mL) were added benzyl 2-phenylpropenate (200 mg, 0.84 mmol) and triethylamine (0.12 mL). The mixture was stirred overnight at room temperature. After addition of water, methanol and acetonitrile were removed under reduced pressure, and the residual mixture was extracted with ethyl acetate. The organic layer was washed with brine and dried over anhydrous sodium sulfate, and the solvent was removed under reduced pressure. The residue was purified by silica gel column chromatography (n-hexane/ethyl acetate=2/1) to give 329 mg (yield 90%) of the titled compound as an orange oil.

¹H-NMR (CDCl₃, 400 MHz) δ: 1.9-2.0 (2H, m), 2.1-2.2 (2H, m), 2.3-2.4 (1H, m), 2.5-2.6 (3H, m), 2.7-2.8 (1H, m), 3.20 (1H, dd, J=11, 13Hz), 3.71 (3H, s), 3.88 (1H, dd, J=5, 11Hz), 4.1-4.2 (1H, br), 5.05 (1H, d, J=12Hz), 5.23 (1H, d, J=12Hz), 6.79 (1H, dd, J=2, 8Hz), 7.2-7.4 (12H, m), 7.56 (1H, dd, J=2, 8Hz).

### (7) Benzyl 3-[4-[3-(tert-butoxycarbonylamino)phenylamino]-4-methoxycarbonylpiperidin-1-yl]-2-phenylpropionate

To a solution of the benzyl 3-[4-methoxycarbonyl-4-(3-nitrophenyl)aminopiperidin-1-yl]-2-phenylpropionate (324 mg, 0.63 mmol) in ethanol (4 mL) was added a solution of calcium chloride (45 mg) in water (1 mL). To the mixture was slowly added zinc powder (1.35 g, 20.6 mmol). The mixture was heated under reflux for 2 hours, and filtered to remove insolubles. The filtrate was evaporated under reduced pressure to give a light brown oil. The oil was dissolved in a mixture of tetrahydrofuran (15 mL) and water (3 mL). To the resulting solution were added triethylamine (0.13 mL) and di-tert-butyl dicarbonate (137 mg, 0.63 mmol). The mixture was stirred for one hour at room temperature, and concentrated under reduced pressure. After addition of water and ethyl acetate, the precipitate was filtered off. The organic layer was washed with brine and dried over anhydrous sodium sulfate, and the solvent was removed under reduced pressure. The residue was purified by silica gel column chromatography (n-hexane/ethyl acetate=2/1) to give 239 mg (yield 64%) of the titled compound.

¹H-NMR (CDCl₃, 400 MHz) δ: 1.50 (9H, s), 1.9-2.7 (9H, m), 3.18 (1H, dd, J=11, 13Hz), 3.67 (3H, s), 3.8-3.9 (2H, m), 5.05 (1H, d, J=12Hz), 5.22 (1H, d, J=12Hz), $6.1-6.2 (1H, m), 6.36 (1H, s), 6.5-6.6 (1H, m), 6.79 (1H, s), 7.01 (1H, t, J=8Hz), 7.2-7.4 (10H, m).

### [Reference Example 3]

### N-(3-Aminophenyl)-N-(4-methoxycarbonylpiperidin-4-yl)propionamide

### (1) Methyl 1-tert-butoxycarbonyl-4-(3-nitrophenyl)aminopiperidine-4-carboxylate

To a solution of methyl 4-(3-nitrophenyl)aminopiperidine-4-carboxylate hydrochloride (140 mg, 0.44 mmol) and triethylamine (0.19 mL) in a mixture of tetrahydrofuran (15 mL) and water (3 mL) was added di-tert-butyl dicarbonate (106 mg, 0.49 mmol). The mixture was then stirred overnight at room temperature. After evaporation of tetrahydrofuran under reduced pressure, the reaction mixture was diluted with ethyl acetate. The organic layer was washed with saturated brine and dried over anhydrous sodium sulfate, and the solvent was removed under reduced pressure. The residue was purified by silica gel column chromatography (n-hexane/ethyl acetate=2/1) to give 162 mg (yield 96%) of the titled compound as a yellow oil.

¹H-NMR (CDCl₃, 400 MHz) δ: 1.48 (9H, s), 1.9-2.0 (2H, m), 2.2-2.3 (2H, m), 3.33 (2H, ddd, J=3, 10, 13Hz), 3.7-3.8 (5H, m), 4.23 (1H, s), 6.87 (1H, dd, J=2, 8Hz), 7.3-7.4 (1H, m), 7.4-7.5 (1H, m), 7.6-7.7 (1H, m).

### (2) Methyl 4-(3-aminophenyl)amino-1-tert-butoxycarbonylpiperidine-4-carboxylate

To a solution of the methyl 1-tert-butoxycarbonyl-4-(3-nitrophenyl)aminopiperidine-4-carboxylate (162 mg, 0.43 mmol) in methanol (2 mL) was added 5% palladium-carbon (34 mg). The resulting mixture was subjected to catalytic hydrogenation (1 atm.) for 6 hours at room temperature. The catalyst was filtered off, and the solvent was removed under reduced pressure to give 136 mg (yield 91%) of the titled compound as a light brown oil.

¹H-NMR (CDCl₃, 400 MHz) δ: 1.44 (9H, s), 1.9-2.0 (2H, m), 2.09 (2H, ddd, J=4, 10, 13Hz), 3.30 (2H, ddd, J=3, 10, 13Hz), 3.47 (3H, s), 3.6-3.8 (5H, m), 5.93 (1H, d, J=2Hz), 6.00 (1H, dd, J=2, 8Hz), 6.12 (1H, dd, J=2, 8Hz), 6.92 (1H, t, J=8Hz).

### (3) Methyl 1-tert-butoxycarbonyl-4-(3-triphenylmethylaminophenyl)aminopiperidine-4-carboxylate

To a solution of the methyl 4-(3-aminophenyl)amino-1-tert-butoxycarbonylpiperidine-4-carboxylate (136 mg, 0.39 mmol) in chloroform (3 mL) were added triethylamine (65 µL) and triphenylmethyl chloride (119 mg, 0.43 mmol). The mixture was stirred for one hour at room temperature. The reaction mixture was diluted with aqueous saturated sodium hydrogencarbonate, and extracted with chloroform. The extract was dried over anhydrous sodium sulfate and the solvent was removed under reduced pressure. The residue was purified by silica gel column chromatography (n-hexane/ethyl acetate=2/1) to give 208 mg (yield 90%) of the titled compound as a white crystal.

¹H-NMR (CDCl₃, 400 MHz) δ: 1.45 (9H, s), 1.5-1.6 (2H, m), 1.80 (2H, ddd, J=4, 10, 14Hz), 3.0-3.1 (2H, m), 3.4-3.6 (6H, m), 4.92 (1H, s), 5.48 (1H, d, J=2Hz), 5.85 (1H, dd, J=2, 8Hz), 5.97 (1H, dd, J=2, 8Hz), 6.76 (1H, t, J=8Hz), 7:2-7.4 (15H, m).

### (4) N-(1-tert-Butoxycarbonyl-4-methoxycarbonylpiperidin-4-yl)-N-(3-triphenylmethylaminophenyl)propionamide

To the methyl 1-tert-butoxycarbonyl-4-(3-triphenylmethylaminophenyl)aminopiperidine-4-carboxylate (180 mg, 0.30 mmol) were added propionic anhydride (0.39 mL, 3.0 mmol) and diisopropylethylamine (0.16 mL, 0.91 mmol). The mixture was stirred at 120°C for 6 hours. The reaction mixture was cooled to room temperature. After addition of aqueous sodium hydrogencarbonate and ethyl acetate, the reaction mixture was stirred overnight at room temperature. The precipitate was collected by filtration and washed with water and n-hexane/ethyl acetate (1/1). The resulting white crystal was dried to give 126 mg (yield 64%) of the titled compound.

¹H-NMR (CDCl₃, 400 MHz) δ: 0.84 (3H, t, J=7Hz), 0.9-2.3 (15H, m), 2.8-3.3 (2H, br), 3.4-3.7 (5H, m), 5.19 (1H, s), 5.9-6.1 (1H, br), 6.4-6.5 (1H, br), 6.65 (1H, dd, J=2, 8Hz), 7.05 (1H, t, J=8Hz), 7.2-7.4 (15H, m).

### (5) N-(3-aminophenyl)-N-(4-methoxycarbonylpiperidin-4-yl)propionamide

The N-(1-tert-butoxycarbonyl-4-methoxycarbonylpiperidin-4-yl)-N-(3-triphenylmethylaminophenyl)propionamide (75 mg, 0.12 mmol) was dissolved in trifluoroacetic acid (1 mL). The resulting solution was stirred for one hour at room temperature. The reaction mixture was placed under reduced pressure to distill trifluoroacetic acid off. The residue was diluted with water and washed with ethyl acetate. The aqueous layer was neutralized with sodium carbonate. After addition of saturated brine, the mixture was extracted with chloroform. The organic layer was dried over anhydrous sodium sulfate and the solvent was removed under reduced pressure to give 29 mg (yield 83%) of the titled compound as a white amorphous.

¹H-NMR (CDCl₃, 400 MHz) δ: 0.96 (3H, t, J=7Hz), 1.4-1.6 (2H, m), 1.8-1.9 (1H, br), 1.97 (2H, q, J=7Hz), 2.2-2.3 (2H, m), 2.8-3.0 (4H, m), 3.79 (3H, s), 3.8-4.0 (2H, br), 6.6-6.7 (3H, m), 7.16 (1H, t, J=8Hz).

### [Reference Example 4]

### Benzyl cis-3-[4-[3-(tert-butoxycarbonylamino)phenylamino]-3-methylpiperidin-1-yl]-2-phenylpropionate

### (1) cis-4-(3-Acetylaminophenyl)amino-1-benzyl-3-methylpiperidine

The procedures of Reference Example 1-(1) were repeated using 1-benzyl-3-methyl-4-piperidone and 3'-aminoacetoanilide, to give the titled compound as a yellow-orange oil. The separation of the cis-form and the trans-form was performed by silica gel column chromatography (ethyl acetate/methanol=25/1).

¹H-NMR (CDCl₃, 400 MHz) δ: 0.97 (3H, d, J=7Hz), 1.6-1.9 (2H, m), 2.0-2.2 (1H, m), 2.14 (3H, s), 2.2-2.4 (3H, m), 2.4-2.6 (1H, m), 3.2-3.6(2H, br), 3.46 (1H, d, J=13Hz), 3.52 (1H, d, J=13Hz), 6.35 (1H, br d, J=8Hz), 6.55 (1H, br d, J=8Hz), 7.07 (1H, t, J=8Hz), 7.1-7.4 (7H, m) .

### (2) cis-4-(3-Aminophenyl) amino-1-benzyl-3-methylpiperidine

The procedures of Reference Example 1-(2) were repeated using the cis-4-(3-Acetylaminophenyl)amino-1-benzyl-3-methylpiperidine, to give the titled compound as a light brown oil.

¹H-NMR (CDCl₃, 400 MHz) δ: 0.96 (3H, d, J=7Hz), 1.6-1.8 (2H, m), 2.1-2.2 (1H, m), 2.2-2.4 (3H, m), 2.4-2.6 (1H, m), 3.2-3.7 (4H, br), 3.44 (1H, d, J=13Hz), 3.52 (1H, d, J=13Hz), 5.95 (1H, t, J=2Hz), 6.03 (1H, dd, J=2, 8Hz), 6.05 (1H, dd, J=2, 8Hz), 6.93 (1H, t, J=8Hz), 7.2-7.4 (5H, m).

### (3) cis-1-Benzyl-4-[3-(tert-butoxycarbonylamino)phenylamino]-3-methylpiperidine

The procedures of Reference Example 1-(3) were repeated using the cis-4-(3-aminophenyl)amino-1-benzyl-3-methylpiperidine to give the titled compound as an orange foam.

¹H-NMR (CDCl₃, 400 MHz) δ: 0.95 (3H, d, J=7Hz), 1.3-1.5 (1H, m), 1.49 (9H, s), 1.6-1.8 (2H, m), 2.1-2.2 (1H, m), 2.2-2.4 (3H, m), 2.4-2.6 (1H, m), 3.44 (1H, d, J=13Hz), 3.51 (1H, d, J=13Hz), 3.5-3.8 (1H, br), 6.27 (1H, dd, J=2, 8Hz), 6.37 (1H, br s), 6.42 (1H, dd, J=2, 8Hz), 6.89 (1H, br s), 7.03 (1H, t, J=8Hz), 7.2-7.4 (5H, m).

### (4) cis-4-[3-(tert-Butoxycarbonylamino)phenylamino]-3-methylpiperidine

The procedures of Reference Example 1-(4) were repeated using the cis-1-benzyl-4-[3-(tert-butoxycarbonylamino)phenylamino]-3-methylpiperidine to give the titled compound as a yellow-brown oil.

¹H-NMR (CDCl₃, 400 MHz) δ: 0.95 (3H, d, J=7Hz), 1.3-1.5 (1H, m), 1.51 (9H, s), 1.67 (2H, q, J=6Hz), 2.0-2.2 (2H, m), 2.7-2.8 (2H, m), 2.85 (1H, dd, J=3, 13Hz), 2.9-3.0 (1H, m), 3.5-3.7 (1H, br), 6.29 (1H, dd, J=2, 8Hz), 6.45 (1H, dd, J=2, 8Hz), 6.55 (1H, br s), 6.91 (1H, br s), 7.04 (1H, t, J=8Hz).

### (5) Benzyl cis-3-[4-[3-(tert-butoxycarbonylamino)phenylamino]-3-methylpiperidin-1-yl]-2-phenylpropionate

The procedures of Reference Example 1-(5) were repeated using the cis-4-[3-(tert-butoxycarbonylamino)-phenylamino]-3-methylpiperidine to give the titled compound as a pale yellow oil.

¹H-NMR (CDCl₃, 400 MHz) δ: 0.85 (1.5H, d, J=7Hz), 0.87 (1.5H, d, J=7Hz), 1.50 (9H, s), 1.5-1.7 (2H, m), 2.0-2.7 (6H, m), 3.1-3.2 (1H, m), 3.4-3.5 (1H, m), 3.5-3.7 (1H, br), 3.8-3.9 (1H, m), 5.05 (0.5H, d, J=13Hz), 5.11 (0.5H, d, J=13Hz), 5.15 (0.5H, d, J=13Hz), 5.20 (0.5H, d, J=13Hz), 6.25 (1H, br d, J=8Hz), 6.36 (1H, br s), 6.43 (1H, br d, J=2Hz), 6.87 (1H, br s), 7.03 (1H, t, J=8Hz), 7.2-7.4 (10H, m).

### [Reference Example 5]

### N-(3-Acetylaminophenyl)-N-(4-methoxycarbonylpiperidin-4-yl)-2-furamide

### (1) N-(1-tert-Butoxycarbonyl-4-methoxycarbonylpiperidin-4-yl)-N-(3-triphenylmethylaminophenyl)-2-furamide

To a suspension of methyl 1-tert-butoxycarbonyl-4-(3-triphenylmethylaminophenyl)aminopiperidine-4-carboxylate (5.92 g, 10 mmol) in dry toluene (100 mL) were added triethylamine (4.18 mL) and 2-furoyl chloride (1.18 mL, 12 mmol). The mixture was heated under reflux for 17 hours. The reaction mixture was cooled to room temperature. The precipitate was collected by filtration, washed with toluene and water, and dried to give 2.86 g (yield 29%) of the titled compound as a white crystal.

¹H-NMR (CDCl₃, 400 MHz) δ: 1.0-1.4 (2H, m), 1.48 (9H, s), 2.8-4.0 (6H, m), 3.62 (3H, s), 5.10 (1H, d, J=3Hz), 5.21 (1H, s), 6.1-6.2 (1H, m), 6.19 (1H, dd, J=1, 3 Hz), 6.5-6.6 (1H, m), 6.6-6.7 (1H, m), 7.06 (1H, t, J=8Hz), 7.1-7.4 (15H, m), 7.40 (1H, br s).

### (2) N-(3-Aminophenyl)-N-(1-tert-butoxycarbonyl-4-methoxycarbonylpiperidin-4-yl)-2-furamide

To a solution of the N-(1-tert-butoxycarbonyl-4-methoxycarbonylpiperidin-4-yl)-N-(3-triphenylmethylaminophenyl)-2-furamide (342 mg, 0.5 mmol) in dichloromethane (14 mL) was added dropwise with stirring under ice-cooling 1%(v/v) trifluoroacetic acid/dichloromethane (20 mL) over one hour. After the addition was complete, the reaction mixture was washed with an aqueous saturated sodium hydrogencarbonate and brine and dried over anhydrous sodium sulfate, and the solvent was removed under reduced pressure. The residue was purified by silica gel column chromatography (chloroform/methanol=40/1) to give 220 mg (yield 99%) of the titled compound as a white amorphous product.

¹H-NMR (CDCl₃, 400 MHz) δ: 1.42 (9H, s), 1.5-1.7 (2H, m), 2.2-2.5 (2H, m), 3.0-3.4 (2H, m), 3.7-4.0 (4H, m), 3.82 (3H, s), 5.41 (1H, d, J=3Hz), 6.16 (1H, dd, J=2, 3Hz), 6.6-6.7.(1H, m), 6.72 (1H, br d, J=8Hz), 6.78 (1H, dd, J=2, 8Hz), 7.21 (1H, t, J=8Hz), 7.38 (1H, br s).

### (3) N-(3-Acetylaminophenyl)-N-(1-tert-butoxycarbonyl-4-methoxycarbonylpiperidin-4-yl)-2-furamide

To a solution of N-(3-aminophenyl)-N-(1-tert-butoxycarbonyl-4-methoxycarbonylpiperidin-4-yl)-2-furamide (220 mg, 0.5 mmol) and pyridine (0.04 mL, 0.5 mmol) in toluene (5 mL) was added acetic anhydride (0.047 mL, 0.5 mmol). The mixture was stirred for 15 hours at room temperature. The reaction mixture was poured into an aqueous sodium hydrogencarbonate and extracted with ethyl acetate. The extract was washed with saturated brine and dried over anhydrous sodium sulfate, and the solvent was removed under reduced pressure. The residue was washed with n-hexane to give 186 mg (yield 77%) of the titled compound as a white powder.

¹H-NMR (CDCl₃, 400 MHz) δ: 1.4-1.7 (2H, m), 1.42 (9H, s), 2.1-2.4 (2H, m), 2.20 (3H, s), 3.1-3.3 (2H, m), 3.7-4.0 (2H, m), 3.80 (3H, s), 5.44 (1H, d, J=3Hz), 6.16 (1H, dd, J=1, 3Hz), 7.09 (1H, br d, J=8Hz), 7.2-7.3 (1H, m), 7.31 (1H, br s), 7.41 (1H, t, J=8Hz), 7.5-8.1 (2H, m).

### (4) N-(3-Acetylaminophenyl)-N-(4-methoxycarbonylpiperidin-4-yl)-2-furamide

To a solution of the N-(3-acetylaminophenyl)-N-(1-tert-butoxycarbonyl-4-methoxycarbonylpiperidin-4-yl)-2-furamide (186 mg, 0.38 mmol) in dichloromethane (2 mL) was added trifluoroacetic acid (0.37 mL). The mixture was stirred for 4 hours at room temperature. The reaction mixture was poured into aqueous sodium hydrogencarbonate. The aqueous mixture was saturated with sodium chloride and extracted four times with chloroform. The extract was dried over anhydrous sodium sulfate and the solvent was removed under reduced pressure to give 146 mg (yield 100%) of the titled compound as a white amorphous product.

¹H-NMR (CDCl₃, 400 MHz) δ: 1.6-1.7 (2H, m), 2.19 (3H, s), 2.2-2.3 (1H, m), 2.4-2.5 (1H, m), 2.9-3.2 (4H, m), 3.81 (3H, s), 5.45 (1H, d, J=3Hz), 6.15 (1H, dd, J=1, 3Hz), 7.12 (1H, d, J=7Hz), 7.3-7.5 (3H, m), 7.79 (1H, br s), 7.91 (1H, d, J=8Hz).

### [Reference Example 6]

### N-(3-Aminophenyl)-N-(4-methoxycarbonylpiperidin-4-yl)-2-furamide

To a suspension of the N-(1-tert-butoxycarbonyl-4-methoxycarbonylpiperidin-4-yl)-N-(3-triphenylmethylaminophenyl)-2-furamide (342 mg, 0.5 mmol) in dichloromethane (7 mL) was added trifluoroacetic acid (0.68 mL). The mixture was stirred for 14 hours at room temperature, and then the solvent was removed under reduced pressure. After addition of water, the residue was washed with ethyl acetate. The aqueous layer was basified by addition of sodium carbonate and saturated with sodium chloride. The aqueous layer was extracted six times with chloroform and dried over anhydrous sodium sulfate, and the solvent was removed under reduced pressure to give 146 mg (yield 85%) of the titled compound as a white crystal.

¹H-NMR (CDCl₃, 400 MHz) δ: 1.5-1.7 (2H, m), 2.3-2.5 (2H, m), 2.9-3.1 (4H, m), 3.78 (2H, s), 3.82 (3H, s), 5.41 (1H, d, J=3Hz), 6.15 (1H, dd, J=1, 3Hz), 6.7-6.8 (3H, m), 7.20 (1H, t, J=8Hz), 7.37 (1H, d, J=1Hz).

### [Reference Example 7]

### N-(3-Acetylaminophenyl)-N-(4-methoxycarbonylpiperidin-4-yl)propionamide

The procedures of Reference Example 5-(2), -(3), and -(4) were repeated using the N-(1-tert-butoxycarbonyl-4-methoxycarbonylpiperidin-4-yl)-N-(3-triphenylmethylaminophenyl)propionamide obtained in Reference Example 3-(4) to give the titled compound as a white amorphous.

¹H-NMR (CD₃OD, 400 MHz) δ: 0.94 (3H, t, J=7Hz), 1.5-1.6 (2H, m), 1.95 (2H, q, J=7Hz), 2.1-2.3 (2H, m), 2.14 (3H, s), 2.7-3.0 (4H, m), 3.77 (3H, s), 7.09 (1H, d, J=8Hz), 7.43 (1H, t, J=8Hz), 7.58 (1H, d, J=8Hz), 7.7-7.8 (1H, m).

### [Reference Example 8]

### 2,4,6-Trimethylbenzyl 3-acetoxy-2-(2-thienyl)-propionate

### (1) 2,4,6-Trimethylbenzyl 3-hydroxy-2-(2-thienyl)-propionate

To a solution of 3-hydroxy-2-(2-thienyl)propionic acid (172 mg, 1.00 mmol) in N,N-dimethylformamide (2 mL) was added potassium hydroxide (79 mg, 1.2 mmol). The mixture was stirred for 1.5 hours at room temperature. To the resulting suspension was added a solution of 2,4,6-trimethylbenzyl chloride (202 mg, 1.20 mmol) in N,N-dimethylformamide (2 mL) under ice-cooling. The mixture was stirred for 30 minutes under ice-cooling and then for 17 hours at room temperature. After addition of cold water, the reaction mixture was extracted with ethyl acetate. The extract was washed with water and saturated brine and dried over anhydrous sodium sulfate, and the solvent was removed under reduced pressure. The residue was purified by silica gel column chromatography (ethyl acetate/n-hexane=1/3) to give 70 mg (yield 23%) of the titled compound as a colorless oil.

¹H-NMR (CDCl₃, 400 MHz) δ: 2.26 (9H, s), 3.8-4.0 (1H, m), 4.0-4.2 (2H, m), 5.17 (1H, d, J=12Hz), 5.25 (1H, d, J=12Hz), 6.84 (2H, s), 6.93 (1H, d, J=2Hz), 6.93 (1H, d, J=4Hz), 7.20 (1H, dd, J=2, 4Hz).

### (2) 2,4,6-Trimethylbenzyl 3-acetoxy-2-(2-thienyl)-propionate

To a solution of the 2,4,6-trimethylbenzyl 3-hydroxy-2-(2-thienyl)propionate (70 mg, 0.23 mmol) in dichloromethane (1 mL) were added pyridine (0.023 mL, 0.28 mmol) and acetic anhydride (0.027 mL, 0.28 mmol). The mixture was stirred for 18 hours at room temperature. The reaction mixture was diluted with chloroform and washed successively with aqueous saturated sodium hydrogencarbonate and brine and dried over anhydrous sodium sulfate, and the solvent was removed under reduced pressure. The residue was purified by silica gel column chromatography (ethyl acetate/n-hexane=1/5) to give 63 mg (yield 78%) of the titled compound as a colorless oil.

¹H-NMR (CDCl₃, 400 MHz) δ: 1.98 (3H, s), 2.27 (3H, s), 2.29 (6H, s), 4.23 (1H, dd, J=6, 9Hz), 4.40 (1H, dd, J=6, 11Hz), 4.50 (1H, dd, J=9, 11Hz), 5.18 (1H, d, J=12Hz), 5.27 (1H, d, J=12Hz), 6.86 (2H, s), 6.9-7.0 (2H, m), 7.23 (1H, dd, J=1, 4Hz).

### [Reference Example 9]

### 4-Methoxybenzyl 3-acetoxy-2-(2-thienyl)propionate

### (1) 4-Methoxybenzyl 3-hydroxy-2-(2-thienyl)propionate

To a solution of 3-hydroxy-2-(2-thienyl)propionic acid (302 mg, 1.75 mmol) in N,N-dimethylformamide (3 mL) was added potassium hydroxide (139 mg, 2.10 mmol). The mixture was stirred for 30 minutes at room temperature. To the resulting suspension was added a solution of 4-methoxybenzyl chloride (329 mg, 2.10 mmol) in N,N-dimethylformamide (2 mL) with stirring under ice-cooling. The mixture was stirred for 30 minutes under ice-cooling and then for 20 hours at room temperature. After addition of cold water, the reaction mixture was extracted with ethyl acetate, washed with saturated brine and dried over anhydrous sodium sulfate, and the solvent was removed under reduced pressure. The residue'was purified by silica gel column chromatography (ethyl acetate/n-hexane=1/2) to give 186 mg (yield 36%) of the titled compound as a yellow oil.

¹H-NMR (CDCl₃, 400 MHz) δ: 3.80 (3H, s), 3.8-4.0 (1H, m), 4.0-4.2 (2H, m), 5.09 (1H, d, J=12Hz), 5.14 (1H, d, J=12Hz), 6.8-7.0 (4H, m), 7.2-7.3 (3H, m).

### (2) 4-Methoxybenzyl 3-acetoxy-2-(2-thienyl)propionate

To a solution of the 4-methoxybenzyl 3-hydroxy-2-(2-thienyl)propionate (186 mg, 0.64 mmol) in dichloromethane (2 mL) were added pyridine (0.062 mL, 0.77 mmol) and acetic anhydride (0.073 mL, 0.77 mmol). The mixture was stirred for 20 hours at room temperature. The reaction mixture was diluted with chloroform, washed successively with aqueous saturated sodium hydrogencarbonate and saturated brine and dried over anhydrous sodium sulfate, and the solvent was removed under reduced pressure. The residue was purified by silica gel column chromatography (ethyl acetate/n-hexane=1/4) to give 163 mg (yield 77%) of the titled compound as a colorless oil.

¹H-NMR (CDCl₃, 400 MHz) δ: 1.97 (3H, s), 3.80 (3H, s), 4.26 (1H, dd, J=6, 9Hz), 4.41 (1H, dd, J=6, 11Hz), 4.53 (1H, dd, J=9, 11Hz), 5.08 (1H, d, J=12Hz), 5.16 (1H, d, J=12Hz), 6. 8-7 . 0 (4H, m), 7.2-7.3 (3H, m).

### [Example 1]

### Benzyl 3-[4-[N-[3-(tert-butoxycarbonyamino)phenyl]-N-(2-furoyl)amino]piperidin-1-yl]-2-phenylpropionate

To a solution of benzyl 3-[4-[3-(tert-butoxycarbonylamino)phenylamino]piperidin-1-yl]-2-phenylpropionate (222 mg, 0.419 mmol) obtained in Reference Example 1 in chloroform (2 mL) were added triethylamine (0.15 mL) and 2-furoyl chloride (50 µL, 0.503 mmol). The mixture was then stirred for 2 hours at room temperature. The reaction mixture was poured into aqueous sodium hydrogencarbonate, and extracted with chloroform. The extract was dried over anhydrous sodium sulfate and the solvent was removed under reduced pressure. The residue was purified by silica gel column chromatography (chloroform/methanol= 99/1) to give 146 mg (yield 56%) of the titled compound as a colorless oil.

¹H-NMR (CDCl₃, 400 MHz) δ: 1.2-1.4 (2H, m), 1.51 (9H, s), 1.7-1.9 (2H, m), 2.14 (1H, br t), 2.31 (1H, br t), 2.50 (1H, dd, J=4, 12Hz), 2.86 (1H, br d), 3.06 (1H, br d), 3.20 (1H, dd, J=10, 12Hz), 3.83 (1H, dd, J=4, 10Hz), 4.6-4.8 (1H, m), 5.04 (1H, d, J=13Hz), 5.13 (1H, d, J=13Hz), 5.57 (1H, d, J=3Hz), 6.15 (1H, dd, J=2, 3 Hz), 6.52 (1H, br s), 6.81 (1H, d, J=8Hz), 7.12 (1H, br s), 7.1-7.4 (12H, m), 7.55 (1H, d, J=8Hz).

### [Example 2]

### Benzyl 3-[4-[N-(3-aminophenyl)-N-(2-furoyl)amino]-piperidin-1-yl]-2-phenylpropionate

To the benzyl 3-[4-[N-[3-(tert-butoxycarbonyamino)-phenyl]-N-(2-furoyl)amino]piperidin-1-yl]-2-phenylpropionate (271 mg, 0.434 mmol) was added trifluoroacetic acid (3 mL). The mixture was then stirred for one hour at room temperature. The reaction mixture was concentrated under reduced pressure. After addition of aqueous sodium hydrogencarbonate, the mixture was extracted with chloroform. The extract was dried over anhydrous sodium sulfate and the solvent was removed under reduced pressure, to give 219 mg (yield 96%) of the titled compound as a white crystal.

¹H-NMR (CDCl₃, 400 MHz) δ: 1.4-1.6 (2H, m), 1.7-1.9 (2H, m), 2.14 (1H, dt, J=2, 12Hz), 2.31 (1H, dt, J=2, 12Hz), 2.51 (1H, dd, J=4, 13Hz), 2.86 (1H, br d), 3.06 (1H, br d), 3.20 (1H, dd, J=11, 13Hz), 3.76 (2H, br s), 3.85 (1H, dd, J=4, 11Hz), 4.6-4.8 (1H, m), 5.04 (1H, d, J=13Hz), 5.13 (1H, d, J=13Hz), 5.54 (1H, d, J=3Hz), 6.15 (1H, dd, J=2, 3Hz), 6.43 (1H, t, J=2Hz), 6.5-6.6 (1H, m), 6.7-6.8 (1H, m), 7.1-7.4 (12H, m).

### [Example 3]

### Benzyl 3-[4-[N-(2-furoyl)-N-(3-ureidophenyl)amino]-piperidin-1-yl]-2-phenylpropionate

To a solution of the benzyl 3-[4-[N-(3-aminophenyl)-N-(2-furoyl)amino]piperidin-1-yl]-2-phenylpropionate (100 mg, 0.191 mmol) in a mixture of acetic acid (0.5 mL) and water (1 mL) was added a solution of sodium cyanate (25 mg, 0.382 mmol) in water (1 mL) at 35°C. The mixture was stirred for 30 minutes. The reaction mixture was poured into the aqueous sodium hydrogencarbonate, and the reaction mixture was extracted with chloroform. The extract was dried over anhydrous sodium sulfate and the solvent was removed under reduced pressure. The residue was purified by silica gel column chromatography (chloroform/methanol=96/4) to give 85 mg (yield 78%) of the titled compound.

¹H-NMR (CDCl₃, 400 MHz) δ: 1.4-1.9 (4H, m), 2.00 (1H, br s), 2.22 (1H, br t), 2.44 (1H, dd, J=4, 12Hz), 2.6-3.2 (2H, br), 3.16 (1H, dd, J=11, 12Hz), 3.81 (1H, dd, J=4, 11Hz), 4.5-4.7 (1H, m), 5.00 (2H, s), 5.03 (1H, d, J=13Hz), 5.14 (1H, d, J=13Hz), 5.44 (1H, br d), 6.12 (1H, dd, J=1, 3Hz), 6.40 (1H, br s), 6.81 (1H, d, J=8Hz), 7.1-7.4 (11H, m), 7.39 (1H, t, J=8Hz), 8.14 (1H, br s), 8.16 (1H, br s).

### [Example 4]

### 3-[4-[N-(2-Furoyl)-N-(3-ureidophenyl)amino]-piperidin-1-yl]-2-phenylpropionic acid

To a solution of the benzyl 3-[4-[N-(2-furoyl)-N-(3-ureidophenyl)amino]piperidin-1-yl]-2-phenylpropionate (80 mg, 0.141 mmol) in a mixture of methanol (4 mL) and tetrahydrofuran (2 mL) was added 10% palladium-carbon (8 mg). The mixture was subjected to catalytic hydrogenation at 1 atm. for 3 hours at room temperature. After filtration of the catalyst, the filtrate was concentrated under reduced pressure. The residue was recrystallized from ethanol, to give the titled compound as a white crystal (59 mg, yield 88%).

¹H-NMR (CD,OD/CDCl₃/D₂O=5/4/1, 400 MHz) δ: 1.7-2.0 (2H, br), 2.0-2.3 (2H, br), 3.0-3.2 (3H, m), 3.5-3.8 (3H, m), 3.83 (1H, dd, J=5, 11Hz), 4.8-5.0 (1H, m), 5.64 (1H, d, J=3Hz), 6.25 (1H, dd, J=1, 3Hz), 6.8-6.9 (1H, m), 7.2-7.6 (9H, m).

IR (cm⁻¹, KBr): 3475, 3027, 1705, 1626, 1599, 1552, 1470, 1373, 1331, 1261, 1232, 1211, 1184, 1134, 1084, 1034, 947, 883, 852, 754, 734, 708, 644, 619, 593, 586, 420.

### [Example 5]

### Benzyl 3-[4-[N-[3-(tert-butoxycarbonyamino)phenyl]-N-propionylamino]piperidin-1-yl]-2-phenylpropionate

To a solution of benzyl 3-[4-[3-(tert-butoxycarbonylamino)phenylamino]piperidin-1-yl]-2-phenylpropionate (222 mg, 0.419 mmol) obtained in Reference Example 1 in chloroform (2 mL) were added triethylamine (0.15 mL) and propionyl chloride (44 µL, 0.503 mmol). The mixture was then stirred for 18 hours at room temperature. After addition of propionyl chloride (36 µL, 0.414 mmol), the mixture was further stirred 2 hours at room temperature, and furthermore, after further addition of propionyl chloride (18 µL, 0.207 mmol), the mixture was stirred 2 hours at room temperature. The reaction mixture was poured into aqueous sodium hydrogencarbonate, and extracted with chloroform. The extract was dried over anhydrous sodium sulfate and the solvent was removed under reduced pressure. The residue was purified by silica gel column chromatography (chloroform/methanol =99/1) to give 235 mg (yield 96%) of the titled compound as a colorless oil.

¹H-NMR (CDCl₃, 400 MHz) δ: 1.01 (3H, t, J=7Hz), 1.2-1.4 (2H, m), 1.52 (9H, s), 1.6-1.8 (2H, m), 1.9-2.0 (2H, m), 2.08 (1H, dt, J=2, 12Hz), 2.25 (1H, dt, J=2, 12Hz), 2.48 (1H, dd, J=4, 13Hz), 2.82 (1H, br d), 3.02 (1H, br d), 3.18 (1H, dd, J=11, 13Hz), 3.83 (1H, dd, J=4, 11Hz), 4.5-4.7 (1H, m), 5.03 (1H, d, J=13Hz), 5.11 (1H, d, J=13Hz), 6.55 (1H, br d), 6.73 (1H, d, J=7Hz), 7.1-7.4 (13H, m).

### [Example 6]

### Benzyl 3-[4-[N-(3-aminophenyl)-N-propionylamino]-piperidin-1-yl]-2-phenylpropionate

The procedures of Example 2 were repeated using the benzyl 3-[4-[N-[3-(tert-butoxycarbonyamino)phenyl]-N-propionylamino]piperidin-1-yl]-2-phenylpropionate to give the titled compound as a colorless oil.

¹H-NMR (CDCl₃, 400 MHz) δ: 1.01 (3H, t, J=7Hz), 1.2-1. 5 (2H, m), 1.6-1.9 (2H, m), 1.99 (2H, q, J=7Hz), 2.08 (1H, br t), 2.25 (1H, br t), 2.48 (1H, dd, J=4, 13Hz), 2.82 (1H, br d), 3.02 (1H, br d), 3.18 (1H, dd, J=11, 13Hz), 3.73 (2H, br s), 3.83 (1H, dd, J=4, 11Hz), 4.5-4.7 (1H, m), 5.03 (1H, d, J=12Hz), 5.12 (1H, d, J=12Hz), 6.36 (1H, br s), 6.44 (1H, d, J=8Hz), 6.69 (1H, dd, J=2, 8Hz), 7.1-7.4 (11H, m).

### [Example 7]

### Benzyl 2-phenyl-3-[4-[N-propionyl-N-(3-ureidophenyl)amino]piperidin-1-yl]propionate

The procedures of Example 3 were repeated using the benzyl 3-[4-[N-(3-aminophenyl)-N-propionylamino]-piperidin-1-yl]-2-phenylpropionate to give the titled compound.

¹H-NMR (CDCl₃, 400 MHz) δ: 1.00 (3H, t, J=8Hz), 1.2-1.5 (2H, m), 1.6-1.8 (2H, m), 1.9-2.1 (3H, m), 2.22 (1H, br t), 2.45 (1H, dd, J=4, 12Hz), 2.7-3.1 (2H, br), 3.17 (1H, t, J=12Hz), 3.83 (1H, dd, J=4, 11Hz), 4.5-4.7 (1H, m), 4.89 (2H, br s), 5.0-5.2 (2H, m), 6.73 (1H, d, J=7Hz), 6.98 (1H, br d), 7.1-7.4 (11H, m), 7.5-7.7 (1H, m), 7.70 (1H, br s).

### [Example 8]

### 2-Phenyl-3-[4-[N-propionyl-N-(3-ureidophenyl)amino]-piperidin-1-yl]propionic acid

The procedures of Example 4 were repeated using the benzyl 2-phenyl-3-[4-[N-propionyl-N-(3-ureidophenyl)-amino]piperidin-1-yl]propionate to give the titled compound as a white powder.

¹H-NMR (CD₃OD, 400 MHz) δ: 1.00 (1H, t, J=8Hz), 1.6-1.8 (2H, br), 1.9-2.2 (4H, m), 2.9-3.2 (3H, m), 3.4-3.6 (2H, m), 3.64 (1H, br d), 3.79 (1H, dd, J=4, 11Hz), 4.6-4.9 (1H, m), 6.82 (1H, d, J=7Hz), 7.1-7.4 (8H, m).

IR (cm⁻¹, KBr): 3336, 1685, 1637, 1593, 1489, 1446, 1350, 1279, 1211, 1103, 793, 721, 702.

### [Example 9]

### Benzyl 3-[4-[N-(2-furoyl)-N-(3-guanidinophenyl)-amino]piperidin-1-yl]-2-phenylpropionate

### (1) Benzyl 3-[4-[N-[3-[2,3-bis(tert-butoxycarbonyl)-guanidino]phenyl]-N-(2-furoyl)amino]piperidin-1-yl]-2-phenylpropionate

To a solution of benzyl 3-[4-[N-(3-aminophenyl)-N-(2-furoyl)amino]piperidin-1-yl]-2-phenylpropionate (82 mg, 0.157 mmol) obtained in Example 2 in a mixture of methanol (3 mL) and tetrahydrofuran (1 mL) were added triethylamine (65 µL, 0.470 mmol), N,N'-bis-tert-butoxycarbonylthiourea (43 mg, 0.157 mmol) and mercury (II) chloride (55 mg, 0.204 mmol). The mixture was stirred for 6 hours at room temperature. After removing the insoluble materials by filtration, the reaction mixture was poured into aqueous sodium hydrogencarbonate and extracted with chloroform. The extract was dried over anhydrous sodium sulfate and the solvent was removed under reduced pressure. The residue was purified by silica gel column chromatography (n-hexane/ethyl acetate=2/1) to give 95 mg (yield 79%) of the titled compound.

¹H-NMR (CDCl₃, 400 MHz) δ: 1.4-1.6 (2H, m), 1.49 (9H, s), 1.54 (9H, s), 1.6-1.8'(2H, m), 2.15 (1H, br t), 2.32 (1H, br t), 2.51 (1H, dd, J=4, 12Hz), 2.86 (1H, br d), 3.04 (1H, br d), 3.20 (1H, dd, J=11, 12Hz), 3.83 (1H, dd, J=4, 11Hz), 4.6-4.8 (1H, m), 5.05 (1H, d, J=13Hz), 5.11 (1H, d, J=13Hz), 5.65 (1H, d, J=3Hz), 6.15 (1H, dd, J=2, 3Hz), 6.88 (1H, d, J=8Hz), 7.1-7.4 (12H, m), 7.48 (1H, br s), 7.80 (1H, d, J=8Hz), 10.38 (1H, s), 11.55 (1H, s).

### (2) Benzyl 3-[4-[N-(2-furoyl)-N-(3-guanidinophenyl)-amino]piperidin-1-yl]-2-phenylpropionate

To the benzyl 3-[4-[N-[3-[2,3-bis-(tert-butoxycarbonyl)guanidino]phenyl]-N-(2-furoyl)amino]piperidin-1-yl]-2-phenylpropionate (120 mg, 0.157 mmol) was added trifluoroacetic acid (2 mL). The mixture was stirred for 2 hours at room temperature. The reaction mixture was concentrated under reduced pressure, and after addition of aqueous sodium hydrogencarbonate, extracted with chloroform. The extract was dried over anhydrous sodium sulfate and the solvent was removed under reduced pressure to give 89 mg (yield 100%) of the titled compound as a colorless oil.

¹H-NMR (CDCl₃, 400 MHz) δ: 1.3-1.5 (2H, m), 1.7-1.9 (2H, m), 2.14 (1H, br t), 2.29 (1H, br t), 2.40 (1H, dd, J=5, 13Hz), 2.87 (1H, br d), 3.04 (1H, br d), 3.18 (1H, dd, J=10, 13Hz), 3.83 (1H, dd, J=5, 10Hz), 4.6-4.8 (1H, m), 5.03 (1H, d, J=13Hz), 5.09 (1H, d, J=13Hz), 6.05 (1H, br s), 6.22 (1H, dd, J=1, 3Hz), 7.02 (1H, br s), 7.12 (1H, d, J=8Hz), 7.1-7.4 (12H, m), 7.47 (1H, t, J=8Hz).

### [Example 10]

### 3-[4-[N-(2-Furoyl)-N-(3-guanidinophenyl)amino]-piperidin-1-yl]-2-phenylpropionic acid

The procedures of Example 4 were repeated using the benzyl 3-[4-[N-(2-furoyl)-N-(3-guanidinophenyl)amino]-piperidin-1-yl]-2-phenylpropionate to give the titled compound as a gray white powder.

¹H-NMR (CD₃OD, 400 MHz) δ: 1.7-2.0 (2H, m), 2.0-2.2 (2H, m), 3.0-3.2 (3H, m), 3.4-3.6 (2H, m), 3.70 (1H, br d), 3.91 (1H, br s), 4.7-5.0 (1H, m), 6.07 (1H, br s), 6.32 (1H, dd, J=2, 3Hz), 7.1-7.6 (10H, m).

IR (cm⁻¹, KBr) : 3325, 1676, 1630, 1599, 1470, 1396, 1369, 1327, 1203, 1188, 1132, 1032, 947, 885, 800, 756, 721, 702, 594.

### [Example 11]

### Benzyl 3-[4-[N-(3-guanidinophenyl)-N-propionylamino]piperidin-1-yl]-2-phenylpropionate

### (1) Benzyl 3-[4-[N-[3-[2,3-bis(tert-butoxycarbonyl)-guanidino]phenyl]-N-propionylamino]piperidin-1-yl]-2-phenylpropionate

The procedures of Example 9-(1) were repeated using benzyl 3-[4-[N-(3-aminophenyl)-N-propionylamino]-piperidin-1-yl]-2-phenylpropionate obtained in Example 6 to give the titled compound.

¹H-NMR (CDCl₃, 400 MHz) δ: 1.02 (1H, t, J=8Hz), 1.2-1.8 (4H, m), 1.49 (9H, s), 1.55 (9H, s), 1.8-2.2 (2H, m), 2.09 (1H, br t), 2.25 (1H, br t), 2.48 (1H, dd, J=4, 12Hz), 2.83 (1H, br d), 3.02 (1H, br d), 3.17 (1H, dd, J=11, 12Hz), 3.7-3.9 (1H, m), 4.5-4.7 (1H, m), 5.03 (1H, d, J=13Hz), 5.10 (1H, d, J=13Hz), 6.82 (1H, d, J=7Hz), 7.1-7.5 (12H, m), 7.62 (1H, d, J=8Hz), 10.36 (1H, s), 11.56 (1H, s).

### (2) Benzyl 3-[4-[N-(3-guanidinophenyl)-N-propionylamino]piperidin-1-yl]-2-phenylpropionate

The procedures of Example 9-(2) were repeated using the benzyl 3-[4-[N-[3-[2,3-bis(tert-butoxycarbonyl)-guanidino]phenyl]-N-propionylaminolpiperidin-1-yl]-2-phenylpropionate to give the titled compound as a colorless oil.

¹H-NMR (CDCl₃, 400 MHz) δ: 1.01 (3H, br t), 1.2-1.4 (2H, m), 1.6-1.8 (2H, m), 1.8-2.2 (2H, m), 2.09 (1H, br t), 2.23 (1H, br t), 2.52 (1H, dd, J=4, 13Hz), 2.83 (1H, br d), 3.00 (1H, br d), 3.15 (1H, dd, J=11, 13Hz), 3.81 (1H, dd, J=4, 11Hz), 4.5-4.7 (1H, m), 5.02 (1H, d, J=13Hz), 5.07 (1H, d, J=13Hz), 6.98 (1H, br s), 7.02 (1H, d, J=8Hz), 7.1-7.4 (11H, m), 7.48 (1H, t, J=8Hz).

### [Example 12]

### 3-[4-[N-(3-Guanidinophenyl)-N-propionylamino]-piperidin-1-yl]-2-phenylpropionic acid

The procedures of Example 4 were repeated using the benzyl 3-[4-[N-(3-guanidinophenyl)-N-propionylamino]-piperidin-1-yl]-2-phenylpropionate to give the titled compound as a white powder.

¹H-NMR (CD₃OD, 400 MHz) δ: 1.00 (3H, t, J=7Hz), 1.5-1.8 (2H, br), 1.9-2.2 (4H, m), 2.9-3.2 (3H, m), 3.4-3.6 (2H, m), 3.66 (1H, br d), 3.83 (1H, dd, J=4, 11Hz), 4.7-5.0 (1H, m), 7.0-7.6 (9H, m).

IR (cm⁻¹, KBr): 3334, 1647, 1597, 1493, 1454, 1379, 1277, 1201, 1136, 800, 721, 702.

### [Example 13]

### Benzyl 3-[4-[N-[3-(tert-butoxycarbonylamino)phenyl]-N-(2-furoyl)amino]-4-methoxycarbonylpiperidin-1-yl]-2-phenylpropionate

To a solution of benzyl 3-[4-[3-(tert-butoxycarbonylamino)phenylamino]-4-methoxycarbonylpiperidin-1-yl]-2-phenylpropionate (238 mg, 0.405 mmol) obtained in Reference Example 2 and triethylamine (0.11 mL) in toluene (2 mL) was added 2-furoyl chloride (60 µL, 0.61 mmol) under ice-cooling. The mixture was stirred at 100°C for 6 hours, and then further stirred overnight at room temperature. After addition of triethylamine (28 µl) and 2-furoyl chloride (20 µl, 0.20 mmol), the mixture was further stirred at 100°C for 2 hours. The reaction mixture was cooled to room temperature, and after addition of aqueous saturated sodium hydrogencarbonate, the organic layer was collected. The aqueous layer was once extracted with ethyl acetate. The combined organic layer was washed with saturated brine, dried over anhydrous sodium sulfate, and the solvent was removed under reduced pressure. The residue was purified by silica gel column chromatography (n-hexane/ethyl acetate=2/1) to give 106 mg (yield 38%) of the titled compound as a white crystal.

¹H-NMR (CDCl₃, 400 MHz) δ: 1.51 (9H, s), 1.6-1.8 (2H, m), 2.2-2.9 (7H, m), 3.19 (1H, t, J=12Hz), 3.78 (3H, s), 3.8-3.9 (1H, m), 5.09 (2H, s), 5.38 (1H, d, J=4Hz), 6.14 (1H, dd, J=2, 4Hz), 6.5-6.6 (1H, m), 7.0-7.1 (1H, m), 7.2-7.4 (12H, m), 7.67 (1H, d, J=8Hz).

### [Example 14]

### Benzyl 3-[4-[N-(3-aminophenyl)-N-(2-furoyl)amino]-4-methoxycarbonylpiperidin-1-yl]-2-phenylpropionate

To the benzyl 3-[4-[N-[3-(tert-butoxycarbonylamino)-phenyl]-N-(2-furoyl)amino]-4-methoxycarbonylpiperidin-1-yl]-2-phenylpropionate (106 mg, 0.155 mmol) was added trifluoroacetic acid (1 mL). The mixture was stirred for one hour at room temperature. To the reaction mixture were added aqueous sodium hydrogencarbonate and chloroform. Then, the organic layer was collected, washed with saturated brine, and dried over anhydrous sodium sulfate, and the solvent was removed under reduced pressure. The residue was purified by silica gel column chromatography (n-hexane/ethyl acetate=1/2) to give 86 mg (yield 96%) of the titled compound.

¹H-NMR (CDCl₃, 400 MHz) δ: 1.6-1.8 (2H, m), 2.1-2.9 (7H, m), 3.20 (1H, t, J=11Hz), 3.76 (2H, s), 3.78 (3H, s), 3.84 (1H, dd, J=4, 11Hz), 5.09 (2H, s), 5.37 (1H, d, J=3Hz), 6.15 (1H, dd, J=2, 3Hz), 6.6-6.8 (3H, m), 7.1-7.4 (12H, m).

### [Example 15]

### 3-[4-[N-(3-Aminophenyl)-N-(2-furoyl)amino]-4-methoxycarbonylpiperidin-1-yl]-2-phenylpropionic acid

The procedures of Example 4 were repeated using the benzyl 3-[4-[N-(3-aminophenyl)-N-(2-furoyl)amino]-4-methoxycarbonylpiperidin-1-yl]-2-phenylpropionate to give the titled compound as a white crystal.

m.p.: 193-195°C (decomp.)

¹H-NMR (CD₃OD, 400 MHz) δ : 1.9-2.2 (2H, m), 2.4-2.6 (2H, m), 3.05 (1H, dd, J=4, 13Hz), 3.1-3.4 (2H, m), 3.4-3.7 (3H, m), 3.82 (3H, s), 3.87 (1H, dd, J=4, 11Hz), 5.51 (1H, d, J=4Hz), 6.27 (1H, dd, J=2, 4Hz), 6.61 (1H, br d), J=8Hz), 6.6-6.7 (1H, m), 6.82 (1H, dd, J=2, 8Hz), 7.16 (1H, t, J=8Hz), 7.2-7.4 (5H, m), 7.53 (1H, d, J=1Hz).

IR (cm⁻¹, KBr): 3477, 3381, 2951, 2858, 1722, 1645, 1628, 1603, 1560, 1491, 1471, 1398, 1356, 1327, 1267, 1234, 1227, 1186, 1146, 1066, 1020, 997, 968, 918, 887, 860, 756, 723, 702, 596, 498.

### [Example 16]

### Benzyl 3-[4-[N-(2-furoyl)-N-(3-ureidophenyl)amino]-4-methoxycarbonylpiperidin-1-yl]-2-phenylpropionate

The procedures of Example 3 were repeated using benzyl 3-[4-[N-(3-aminophenyl)-N-(2-furoyl)amino]-4-methoxycarbonylpiperidin-1-yl]-2-phenylpropionate obtained in Example 14 to give the titled compound as a white crystal.

¹H-NMR (CDCl₃, 400 MHz) δ: 1.5-1.7 (1H, m), 2.1-2.9 (8H, m), 3.16 (1H, dd, J=11, 12Hz), 3.68 (3H, s), 3.7-3.9 (1H, m), 4.9-5.2 (4H, m), 5.31 (1H, dd, J=2, 4Hz), 6.12 (1H, dd, J=2, 4Hz), 6.5-6.7 (1H, m), 6.99 (1H, d, J=8Hz), 7.1-7.4 (11H, m), 7.41 (1H, dd, J=8, 13Hz), 7.85 (1H, s), 8.2-8.3 (1H, m).

### [Example 17]

### 3-[4-[N-(2-Furoyl)-N-(3-ureidophenyl)amino]-4-methoxycarbonylpiperidin-1-yl]-2-phenylpropionic acid

The procedures of Example 4 were repeated using the benzyl 3-[4-[N-(2-furoyl)-N-(3-ureidophenyl)amino]-4-methoxycarbonylpiperidin-1-yl]-2-phenylpropionate to give the titled compound as a white crystal.

m.p.: 170-172°C (decomp.)

¹H-NMR (CDCl₃/CD₃OD/D₂O=5/4/1, 400 MHz) δ: 2.0-2.3 (2H, m), 2.3-2.5 (1H, m), 2.7-2.9 (1H, m), 3.0-3.3 (3H, m), 3.3-3.7 (3H, m), 3.84 (3H, s), 3.8-3.9 (1H, m), 5.48 (1H, d, J=4Hz), 6.24 (1H, dd, J=2, 4Hz), 7.01 (1H, d, J=7Hz), 7.2-7.4 (6H, m), 7.46 (1H, d, J=2Hz), 7.4-7.6 (2H, m).

IR (cm⁻¹, KBr): 3467, 1740, 1686, 1637, 1593, 1560, 1491, 1471, 1450, 1423, 1398, 1356, 1254, 1219, 1184, 11440, 1030, 1009, 976, 949, 914, 885, 856, 754, 723, 702, 627, 594.

### [Example 18]

### Benzyl 3-[4-[N-(3-acetylaminophenyl)-N-(2-furoyl)-amino]-4-methoxycarbonylpiperidin-1-yl]-2-phenylpropionate

To a solution of benzyl 3-[4-[N-(3-aminophenyl)-N-(2-furoyl)amino]-4-methoxycarbonylpiperidin-1-yl]-2-phenylpropionate (1.28 g, 2.20 mmol) obtained in Example 14 in toluene (13 mL) were added triethylamine (0.4 mL) and acetic anhydride (0.21 mL, 2.20 mmol). The mixture was stirred for 18 hours at room temperature. The reaction mixture was poured into aqueous sodium hydrogencarbonate and extracted with chloroform. The extract was dried over anhydrous sodium sulfate and the solvent was removed under reduced pressure. The residue was purified by silica gel column chromatography (n-hexane/ethyl acetate=1/3) to give 1.04 g (yield 76%) of the titled compound as a white crystal.

¹H-NMR (CDCl₃, 400 MHz) δ: 1.5-1.7 (2H, m), 1.9-2.8 (7H, m), 2.17 (3H, s), 3.1-3.3 (1H, m), 3.77 (3H, s), 3.82 (1H, dd, J=4, 11Hz), 5.09 (2H, s), 5.40 (1H, d, J=3Hz), 6.14 (1H, dd, J=1, 3Hz), 7.11 (1H, d, J=8Hz), 7.2-7.4 (12H, m), 7.40 (1H, t, J=8Hz), 7.69 (1H, br d), 7.98 (1H, d, J=8Hz) .

### [Example 19]

### 3-[4-[N-(3-Acetylaminophenyl)-N-(2-furoyl)amino]-4-methoxycarbonylpiperidin-1-yl]-2-phenylpropionic acid

The procedures of Example 4 were repeated using the benzyl 3-[4-[N-(3-acetylaminophenyl)-N-(2-furoyl)amino]-4-methoxycarbonylpiperidin-1-yl]-2-phenylpropionate to give the titled compound as a white crystal.

m.p.: 172-177°C (decomp.)

¹H-NMR (CD₃OD, 400 MHz) δ: 1.9-2.2 (2H, m), 2.11 (3H, s), 2.45 (1H, br t), 2.64 (1H, br t), 3.06 (1H, br d), 3.1-3.7 (5H, m), 3.82 (3H, s), 3.8-3.9 (1H, m), 5.56 (1H, d, J=3Hz), 6.27 (1H, dd, J=1, 3Hz), 7.13 (1H, d, J=8Hz), 7.1-7.5 (6H, m), 7.50 (1H, d, J=1Hz), 7.69 (1H, d, J=8Hz), 7.77 (1H, br s).

IR (cm⁻¹, KBr): 3280, 1740, 1689, 1603, 1552, 1470, 1433, 1404, 1363, 1317, 1296, 1277, 1257, 1217, 1180, 1144, 1090, 1038, 960, 868, 764, 723, 704, 629.

### [Example 20]

### Benzyl 3-[4-[N-(2-furoyl)-N-(3-guanidinophenyl)-amino]-4-methoxycarbonylpiperidin-1-yl]-2-phenylpropionate

### (1) Benzyl 3-[4-[N-[3-[2,3-bis(tert-butoxycarbonyl)-guanidino]phenyl]-N-(2-furoyl)amino]-4-methoxycarbonylpiperidin-1-yl]-2-phenylpropionate

The procedures of Example 9-(1) were repeated using benzyl 3-[4-[N-(3-aminophenyl)-N-(2-furoyl)amino]-4-methoxycarbonylpiperidin-1-yl]-2-phenylpropionate obtained in Example 14, to give the titled compound.

¹H-NMR (CDCl₃, 400 MHz) δ: 1.4-1.6 (18H, m), 1.6-1.8 (2H, m), 2.2-2.9 (7H, m), 3.1-3.3 (1H, m), 3.79 (3H, s), 3.7-3.9 (1H, m), 5.08 (2H, s), 5.49 (1H, s), 6. 15 (1H, dd, J=2, 3Hz), 7.0-7.7 (14H, m), 7.8-8.0 (1H, m), 10.39 (1H, s), 11.5-11.6 (1H, br).

### (2) Benzyl 3-[4-[N-(2-furoyl)-N-(3-guanidinophenyl)-amino]-4-methoxycarbonylpiperidin-1-yl]-2-phenylpropionate

The procedures of Example 9-(2) were repeated using the benzyl 3-[4-[N-[3-[2,3-bis(tert-butoxycarbonyl)-guanidino]phenyl]-N-(2-furoyl)amino]-4-methoxycarbonylpiperidin-1-yl]-2-phenylpropionate to give the titled compound.

¹H-NMR (CDCl₃, 400 MHz) δ: 1.6-1.8 (2H, m), 2.2-2.7 (6H, m), 2.81 (1H, d, J=11Hz), 3.26 (1H, t, J=11Hz), 3.78 (3H, s), 3.8-3.9 (1H, m), 5.08 (2H, s), 5.44 (1H, d, J=3Hz), 6.16 (1H, q, J=2Hz), 6.95 (1H, s), 7.0-7.4 (14H, m).

### [Example 21]

### 3-[4-[N-(2-Furoyl)-N-(3-guanidinophenyl)amino]-4-methoxycarbonylpiperidin-1-yl]-2-phenylpropionic acid

The procedures of Example 4 were repeated using the benzyl 3-[4-[N-(2-furoyl)-N-(3-guanidinophenyl)amino]-4-methoxycarbonylpiperidin-1-yl]-2-phenylpropionate to give the titled compound as a pale yellow powder.

¹H-NMR (CD₃OD, 400 MHz) δ: 1.7-3.4 (10H, m), 3.77 (3H, s), 3.6-3.8 (1H, m), 5.72 (1H, br s), 6.26 (1H, dd, J=1, 3Hz), 7.1-7.6 (10H, m).

### [Example 22]

### Benzyl 3-[4-[N-(3-aminophenyl)-N-propionylamino]-4-methoxycarbonylpiperidin-1-yl]-2-phenylpropionate

To a solution of N-(3-aminophenyl)-N-(4-methoxycarbonylpiperidin-4-yl)propionamide (29.3 g, 0.096 mmol) obtained in Reference Example 3 in a mixture of acetonitrile (300 mL) and methanol (100 mL) were added triethylamine (27 mL, 0.192 mol) and benzyl 2-phenylpropenate (22.9 g, 0.096 mol). The mixture was stirred for 41 hours at room temperature. The reaction mixture was then concentrated under reduced pressure. The residue was purified by silica gel column chromatography (n-hexane/ethyl acetate=1/2) to give 41.3 g (yield 65%) of the titled compound as a white foam.

¹H-NMR (CDCl₃, 400 MHz) δ: 0.96 (3H, t, J=7Hz), 1.5-1.7 (2H, m), 1.95 (2H, q, J=7Hz), 2.1-2.6 (6H, m), 2.6-2.8 (1H, m), 3.1-3.3 (1H, m), 3.74 (2H, s), 3.76 (3H, s), 3.83 (1H, dd, J=4, 11Hz), 5.06 (1H, d, J=13Hz), 5.10 (1H, d, J=13Hz), 6.5-6.8 (3H, m), 7.15 (1H, t, J=8Hz), 7.2-7.4 (10H, m).

### [Example 23]

### 3-[4-[N-(3-Aminophenyl)-N-propionylamino]]-4-methoxycarbonylpiperidin-1-yl]-2-phenylpropionic acid

The procedures of Example 4 were repeated using the benzyl 3-[4-[N-(3-aminophenyl)-N-propionylamino]]-4-methoxycarbonylpiperidin-1-yl]-2-phenylpropionate to give the titled compound as a white crystal.

m.p.: 130-134°C

¹H-NMR (CD₃OD, 400 MHz) δ: 0.95 (3H, t, J=7Hz), 1.8-2.0 (2H, m), 2.02 (2H, q, J=7Hz), 2.4-2.6 (2H, m), 3.0-3.6 (6H, m), 3.78 (3H, s), 3.7-3.9 (1H, m), 6.5-6.7 (1H, m), 6.66 (1H, br s), 6.7-6.8 (1H, m), 7.1-7.4 (6H, m) .

IR (cm⁻¹, KBr) : 3427, 3246, 1734, 1635, 1603, 1491, 1452, 1381, 1238, 1221, 1142, 1068, 997, 953, 864, 729, 702.

### [Example 24]

### Benzyl 3-[4-methoxycarbonyl-4-[N-propionyl-N-(3-ureidophenyl)amino]piperidin-1-yl]-2-phenylpropionate

The procedures of Example 3 were repeated using benzyl 3-[4-[N-(3-aminophenyl)-N-propionylamino]-4-methoxycarbonylpiperidin-1-yl]-2-phenylpropionate obtained in Example 22 to give the titled compound.

¹H-NMR (CDCl₃, 400 MHz) δ: 0.93 (3H, t, J=7Hz), 1.5-1.7 (2H, m), 1.8-2.0 (3H, m), 2.0-2.6 (5H, m), 2.6-2.8 (1H, m), 3.1-3.2 (1H, m), 3.73 (3H, s), 3.83 (1H, dd, J=4, 10Hz), 5.0-5.1 (2H, m), 5.15 (2H, br s), 6.8-6.9 (1H, m), 7.2-7.4 (12H, m), 7.7-7.8 (1H, m), 7.76 (1H, br s).

### [Example 25]

### 3-[4-Methoxycarbonyl-4-[N-propionyl-N-(3-ureido-phenyl)amino]piperidin-1-yl]-2-phenylpropionic acid

The procedures of Example 4 were repeated using the benzyl 3-[4-methoxycarbonyl-4-[N-propionyl-N-(3-ureido-phenyl)amino]piperidin-1-yl]-2-phenylpropionate to give the titled compound as a white crystal.

m.p.: 162-168°C

¹H-NMR (CD₃OD, 400 MHz) δ: 0.94 (3H, t, J=7Hz), 1.8-2.1 (4H, m), 2.3-2.6 (2H, m), 3.0-3.1 (1H, m), 3.1-3.6 (5H, m), 3.79 (3H, s), 3.7-3.9 (1H, m), 6.9-7.0 (1H, m), 7.2-7.4 (7H, m), 7.5-7.6 (1H, m).

IR (cm⁻¹, KBr) : 3427, 1724, 1689, 1624, 1593, 1551, 1483, 1439, 1394, 1354, 1292, 1236, 1213, 1184, 1144, 1115, 1082, 997, 955, 864, 791, 750, 725, 708, 667, 559.

### [Example 26]

### Benzyl 3-[4-[N-(3-acetylaminophenyl)-N-propionylamino]-4-methoxycarbonylpiperidin-1-yl]-2-phenylpropionate

The procedures of Example 18 were repeated using benzyl 3-[4-[N-(3-aminophenyl)-N-propionylamino]-4-methoxycarbonylpiperidin-1-yl]-2-phenylpropionate obtained in Example 22 to give the titled compound.

¹H-NMR (CDCl₃, 400 MHz) δ: 0.95 (3H, t, J=7Hz), 1.5-1.7 (2H, m), 1.8-2.0 (2H, m), 2.16 (3H, s), 2.1-2.3 (2H, m), 2.3-2.6 (4H, m), 2.6-2.8 (1H, m), 3.1-3.2 (1H, m), 3.75 (3H, s), 3.82 (1H, dd, J=4, 11Hz), 5.0-5.2 (2H, m), 7.0-7.1 (1H, m), 7.1-7.3 (10H, m), 7.35 (1H, t, J=8Hz), 7.46 (1H, br s), 7.6-7.7 (1H, m), 7.7-7.9 (1H, m).

### [Example 27]

### 3-[4-[N-(3-Acetylaminophenyl)-N-propionylamino]-4-methoxycarbonylpiperidin-1-yl]-2-phenylpropionic acid

The procedures of Example 4 were repeated using the benzyl 3-[4-[N-(3-acetylaminophenyl)-N-propionylamino]-4-methoxycarbonylpiperidin-1-yl]-2-phenylpropionate to give the titled compound as a white crystal.

m.p.: 141-144°C

¹H-NMR (CD₃OD, 400 MHz) δ: 0.94 (3H, t, J=7Hz), 1.9-2.1 (4H, m), 2.13 (3H, s), 2.2-2.4 (1H, m), 2.5-2.7 (1H, m), 2.9-3.7 (6H, m), 3.80 (3H, s), 3.83 (1H, dd, J=4, 11Hz), 7.00 (1H, br d, J=8Hz), 7.2-7.4 (6H, m), 7.54 (1H, br d, J=8Hz), 7.74 (1H, br s).

IR (cm⁻¹, KBr): 3321, 3290, 2993, 2956, 1740, 1697, 1639, 1596, 1556, 1483, 1452, 1427, 1406, 1367, 1317, 1284, 1257, 1215, 1173, 1144, 1086, 1063, 1001, 960, 908, 862, 795, 779, 704, 685, 629, 580, 536, 445, 415.

### [Example 28]

### Benzyl 3-[4-[N-(3-guanidinophenyl)-N-propionylamino]-4-methoxycarbonylpiperidin-1-yl]-2-phenylpropionate

### (1) Benzyl 3-[4-[N-[3-[2,3-bis(tert-butoxycarbonyl)-guanidino]phenyl]-N-propionylamino]-4-methoxycarbonylpiperidin-1-yl]-2-phenylpropionate

The procedures of Example 9-(1) were repeated using benzyl 3-[4-[N-(3-aminophenyl)-N-propionylamino]-4-methoxycarbonylpiperidin-1-yl]-2-phenylpropionate obtained in Example 22 to give the titled compound.

¹H-NMR (CDCl₃, 400 MHz) δ: 0.97 (3H, t, J=7Hz), 1.4-1.7 (20H, m), 1.8-2.0 (2H, m), 2.1-2.8 (7H, m), 3.1-3.2 (1H, m), 3.77 (3H, s), 3.7-3.9 (1H, m), 5.05 (1H, d, J=12Hz), 5.09 (1H, d, J=12Hz), 7.04 (1H, t, J=7Hz), 7.1-7.3 (10H, m), 7.36 (1H, t, J=8Hz), 7.5-7.6 (1H, m), 7.6-7.8 (1H, m), 10.36 (1H, s), 11.56 (1H, br s).

### (2) Benzyl 3-[4-[N-(3-guanidinophenyl)-N-propionylamino]-4-methoxycarbonylpiperidin-1-yl]-2-phenylpropionate

The procedures of Example 9-(2) were repeated using the benzyl 3-[4-[N-[3-(2,3-bis(tert-butoxycarbonyl)-guanidino]phenyl]-N-propionylamino]-4-methoxycarbonylpiperidin-1-yl]-2-phenylpropionate to give the titled compound.

¹H-NMR (CDCl₃, 400 MHz) δ: 0.93 (3H, t, J=7Hz), 1.4-1.6 (2H, m), 1.87 (2H, q, J=7Hz), 2.1-2.3 (2H, m), 2.3-2.8 (5H, m), 3.0-3.2 (1H, m), 3.74 (3H, s), 3.7-3.9 (1H, m), 5.0-5.2 (2H, m), 5.8-6.4 (4H, br), 7.1-7.4 (13H, m), 7.44 (1H, t, J=8Hz).

### [Example 29]

### 3-[4-[N-(3-Guanidinophenyl)-N-propionylamino]-4-methoxycarbonylpiperidin-1-yl]-2-phenylpropionic acid

The procedures of Example 4 were repeated using the benzyl 3-[4-[N-(3-guanidinophenyl)-N-propionylamino]-4-methoxycarbonylpiperidin-1-yl]-2-phenylpropionate to give the titled compound as a white powder.

¹H-NMR (CD₃OD, 400 MHz) δ: 0.95 (3H, t, J=7Hz), 1.7-2.1 (4H, m), 2.2-2.4 (1H, m), 2.5-2.6 (1H, m), 2.8-3.5 (6H, m), 3.80 (3H, s), 3.7-3.9 (1H, m), 7.1-7.5 (8H, m), 7.60 (1H, t, J=8Hz).

### [Example 30]

### Benzyl cis-3-[4-[N-(3-aminophenyl)-N-(2-furoyl)-amino]-3-methylpiperidin-1-yl]-2-phenylpropionate

### (1) Benzyl cis-3-[4-[N-[3-(tert-butoxycarbonylamino)-phenyl]-N-(2-furoyl)amino]-3-methylpiperidin-1-yl]-2-phenylpropionate

The procedures of Example 1 were repeated using benzyl cis-3-[4-[3-(tert-butoxycarbonylamino)phenylamino]-3-methylpiperidin-1-yl]-2-phenylpropionate obtained in Reference Example 4 to give the titled compound as a yellow oil.

### (2) Benzyl cis-3-[4-[N-(3-aminophenyl)-N-(2-furoyl)-amino]-3-methylpiperidin-1-yl]-2-phenylpropionate

To the benzyl cis-3-[4-[N-[3-(tert-butoxycarbonylamino)phenyl]-N-(2-furoyl)amino]-3-methylpiperidin-1-yl]-2-phenylpropionate (340 mg, 0.533 mmol) was added 4M hydrogen chloride/dioxane (3 mL). The mixture was stirred for 2 hours at room temperature. After addition of aqueous sodium carbonate, the reaction mixture was extracted with ethyl acetate. The extract was washed with water and brine and dried over anhydrous sodium sulfate, and the solvent was removed under reduced pressure to give 284 mg (yield 99%) of the titled compound as a pale yellow-brown powder.

¹H-NMR (CDCl₃, 400 MHz) δ: 1.03 (3H, d, J=7Hz), 1.1-1.3 (1.5H, m), 1.5-1.7 (0.5H, m), 1.9-2.2 (1H, m), 2.29 (0.5H, dd, J=3, 12Hz), 2.42 (0.5H, dt, J=5, 13Hz), 2.4-2.6 (1H, m), 2.6-3.0 (3H, m), 3.07 (0.5H, dd, J=11, 13Hz), 3.12 (0.5H, t, J=12Hz), 3.72 (2H, br s), 3.8-3.9 (1H, m), 4.4-4.5 (1H, m), 5.01 (0.5H, d, J=13Hz), 5.07 (0.5H, d, J=13Hz), 5.15 (0.5H, d, J=13Hz), 5.18 (0.5H, d, J=13Hz), 5.59 (0.5H, d, J=3Hz), 5.60 (0.5H, d, J=3Hz), 6.1-6.2 (1H, m), 6.2-6.8 (3H, m), 7.0-7.4 (12H, m).

### [Example 31]

### Benzyl cis-3-[4-[N-(2-furoyl)-N-(3-ureidophenyl)-amino]-3-methylpiperidin-1-yl]-2-phenylpropionate

The procedures of Example 3 were repeated using benzyl cis-3-[4-[N-(3-aminophenyl)-N-(2-furoyl)amino]-3-methylpiperidin-1-yl]-2-phenylpropionate to give the titled compound as a pale yellow powder.

¹H-NMR (CDCl₃, 400 MHz) δ: 0.8-1.1 (3H, br), 1.4-1.7 (2H, m), 1.8-2.5 (3H, m), 2.5-3.2 (4H, m), 3.7-3.9 (1H, m), 4.3-4.5 (1H, br), 4.91 (1H, br s), 4.93 (1H, br s), 4.98 (0.5H, d, J=13Hz), 5.05 (0.5H, d, J=13Hz), 5.14 (0.5H, d, J=13Hz), 5.17 (0.5H, d, J=13Hz), 5.48 (0.5H, d, J=3Hz), 5.49 (0.5H, d, J=3Hz), 6.1-6.2 (1H, m), 6.3-7.2 (2H, br), 7.2-7.4 (12H, m), 7.85 (1H, br s), 7.9-8.0 (1H, m).

### [Example 32]

### cis-3-[4-[N-(2-Furoyl)-N-(3-ureidophenyl)amino]-3-methylpiperidin-1-yl]-2-phenylpropionic acid

The procedures of Example 4 were repeated using the benzyl cis-3-[4-[N-(2-furoyl)-N-(3-ureidophenyl)amino]-3-methylpiperidin-1-yl]-2-phenylpropionate to give the titled compound as a white powder.

¹H-NMR (CD₃OD/CDCl₃/D₂O=4/1/2, 400 MHz) δ: 1.2-1.4 (3H, m), 1.6-2.1 (2H, m), 3.0-3.7 (7H, m), 3.8-4.0 (1H, m), 4.6-4.8 (1H, m), 5.67 (1H, br s), 6.30 (0.5H, d, J=3Hz), 6.32 (0.5H, d, J=3Hz), 6.8-7.8 (10H, m).

### [Example 33]

### 2,4,6-Trimethylbenzyl 3-[4-[N-(3-acetylaminophenyl)-N-propionylamino]-4-methoxycarbonylpiperidin-1-yl]-2-(2-thienyl)propionate

To a solution of N-(3-acetylaminophenyl)-N-(4-methoxycarbonylpiperidin-4-yl)propionamide (63 mg, 0.18 mmol) obtained in Reference Example 7 and triethylamine (18 mg, 0.18 mmol) in dichloromethane (1 mL) was added a solution of 2,4,6-trimethylbenzyl 3-acetoxy-2-(2-thienyl)propionate (63 mg, 0.18 mmol) obtained in Reference Example 8 in dichloromethane (1 mL). The mixture was stirred for 21 hours at room temperature. The reaction mixture was diluted with chloroform, washed with saturated brine and dried over anhydrous sodium sulfate, and the solvent was removed under reduced pressure. The residue was purified by silica gel column chromatography (ethyl acetate) to 39 mg (yield 34%) of the titled compound as a colorless oil.

¹H-NMR (CDCl₃, 400 MHz) δ: 0.96 (3H, t, J=7Hz), 1.5-1.7 (2H, m), 1.8-2.0 (2H, m), 2.0-2.6 (6H, m), 2.19 (3H, s), 2.22 (3H, s), 2.23 (3H, s), 2.26 (3H, s), 2.6-2.8 (1H, m), 3.0-3.1 (1H, m), 3.77 (3H, s), 4.0-4.1 (1H, m), 5.0-5.2 (2H, m), 6.83 (2H, s), 6.8-6.9 (2H, m), 7.04 (1H, br d, J=7Hz), 7.16 (1H, d, J=5Hz), 7.3-7.4 (2H, m), 7.48 (1H, br s), 7.6-7.7 (1H, m).

### [Example 34]

### 3-[4-[N-(3-Acetylaminophenyl)-N-propionylamino]-4-methoxycarbonylpiperidin-1-yl]-2-(2-thienyl)propionic acid

To the 2,4,6-trimethylbenzyl 3-[4-[N-(3-acetylaminophenyl)-N-propionylamino]-4-methoxycarbonylpiperidin-1-yl]-2-(2-thienyl)propionate (39 mg, 0.062 mmol) was added trifluoroacetic acid (0.4 mL). The mixture was stirred for one hour at room temperature. The reaction mixture was concentrated under reduced pressure. The residue was purified by silica gel column chromatography (chloroform/methanol=5/1) to give 25 mg (yield 81%) of the titled compound as a white powder.

¹H-NMR (CD₃OD, 400 MHz) δ: 0.94 (3H, t, J=8Hz), 1.9-2.0 (4H, m), 2.13 (3H, s), 2.3-2.4 (1H, m), 2.5-2.6 (1H, m), 3.1-3.4 (4H, m), 3.4-3.6 (2H, m), 3.80 (3H, s), 4.1-4.2 (1H, m), 6.9-7.0 (2H, m), 7.05 (1H, t, J=7Hz), 7.29 (1H, d, J=5Hz), 7.3-7.4 (1H, m), 7.54 (1H, br d, J=8Hz), 7.76 (1H, d, J=1Hz).

IR (cm⁻¹, KBr): 3431, 3313, 2951, 1736, 1660, 1599, 1552, 1487, 1433, 1371, 1255, 1142, 1086, 1001, 951, 798, 752, 719.

### [Example 35]

### 4-Methoxybenzyl 3-[4-[N-(3-acetylaminophenyl)-N-(2-furoyl)amino]-4-methoxycarbonylpiperidin-1-yl]-2-(2-thienyl)propionate

To a solution of N-(3-acetylaminophenyl)-N-(4-methoxycarbonylpiperidin-4-yl)-2-furamide (146 mg, 0.38 mmol) obtained in Reference Example 5 and triethylamine (38 mg, 0.38 mmol) in dichloromethane (2 mL) was added a solution of 4-methoxybenzyl 3-acetoxy-2-(2-thienyl)-propionate (127 mg, 0.38 mmol) obtained in Reference Example 9 in dichloromethane (2 mL). The mixture was stirred for 14 hours at room temperature. The reaction mixture was diluted with chloroform, washed with aqueous saturated sodium hydrogencarbonate and saturated brine, and dried over anhydrous sodium sulfate, and the solvent was removed under reduced pressure. The residue was purified by silica gel column chromatography (chloroform/methanol=20/1) to give 60 mg (yield 24%) of the titled compound as a colorless oil.

¹H-NMR (CDCl₃, 400 MHz) δ: 1.5-1.7 (2H, m), 2.1-2.8 (7H, m), 2.18 (3H, s), 3.0-3.2 (1H, m), 3.79 (3H, s), 3.80 (3H, s), 4.0-4.2 (1H, m), 5.0-5.1 (2H, m), 5.42 (1H, d, J=3Hz), 6.15 (1H, dd, J=1, 3Hz), 6.8-7.0 (4H, m), 7.1-7.5 (8H, m), 7.93 (1H, d, J=8Hz).

### [Example 36]

### 3-[4-[N-(3-Acetylaminophenyl)-N-(2-furoyl)amino]-4-methoxycarbonylpiperidin-1-yl]-2-(2-thienyl)propionic acid

To the 4-methoxybenzyl 3-[4-[N-(3-acetylaminophenyl)-N-(2-furoyl)amino]-4-methoxycarbonylpiperidin-1-yl]-2-(2-thienyl)propionate (105 mg, 0.16 mmol) was added trifluoroacetic acid (0.5 mL). The mixture was stirred for one hour under ice-cooling. The reaction mixture was concentrated under reduced pressure. The residue was purified by silica gel column chromatography (chloroform/methanol=5/1) to give 75 mg (yield 88%) of the titled compound as a white powder.

¹H-NMR (CD₃OD, 400 MHz) δ: 2.0-2.2 (2H, m), 2.13 (3H, s), 2.4-2.7 (2H, m), 3.2-3.6 (5H, m), 3.62 (1H, dd, J=11, 12Hz), 3.83 (3H, s), 4.2-4.3 (1H, m), 5.58. (1H, d, J=3Hz), 6.28 (1H, dd, J=1, 3Hz), 6.97 (1H, dd, J=4, 5Hz), 7.01 (1H, d, J=4Hz), 7.16 (1H, br d, J=8Hz), 7.32 (1H, d, J=5Hz), 7.4-7.5 (1H, m), 7.51 (1H, d, J=1Hz), 7.66 (1H, br d, J=7Hz), 7.81 (1H, br s).

IR (cm⁻¹, KBr): 3421, 2954, 1736, 1676, 1599, 1560, 1470, 1433, 1396, 1323, 1248, 1203, 1184, 1138, 1092, 1036, 1014, 951, 837, 800, 764, 721.

### [Example 37]

### 4-Methoxybenzyl 3-[4-[N-(3-aminophenyl)-N-(2-furoyl)amino]-4-methoxycarbonylpiperidin-1-yl]-2-(2-thienyl)propionate

The procedures of Example 35 were repeated using N-(3-aminophenyl)-N-(4-methoxycarbonylpiperidin-4-yl)-2-furamide obtained in Reference Example 6 to give the titled compound as a colorless oil.

¹H-NMR (CDCl₃, 400 MHz) δ: 1.6-1.8 (2H, m), 2.2-2.6 (6H, m), 2.62 (1H, dd, J=5, 12Hz), 2.7-2.9 (1H, m), 3.11 (1H, dd, J=11, 12Hz), 3.78 (3H, s), 3.80 (3H, s), 3.7-3.9 (2H, br), 4.11 (1H, dd, J=5, 11Hz), 5.0-5.1 (2H, m), 5.38 (1H, d, J=3Hz), 6.15 (1H, dd, J=1, 3Hz), 6.6-7.0 (7H, m), 7.1-7.3 (4H, m), 7.37 (1H, d, J=1Hz).

### [Example 38]

### 3-[4-[N-(3-Aminophenyl)-N-(2-furoyl)amino]-4-methoxycarbonylpiperidin-1-yl]-2-(2-thienyl)propionic acid

The procedures of Example 36 were repeated using the 4-methoxybenzyl 3-[4-[N-(3-aminophenyl)-N-(2-furoyl)-amino]-4-methoxycarbonylpiperidin-1-yl]-2-(2-thienyl)propionate to give the titled compound as a pale yellow powder.

¹H-NMR (CD₃OD, 400 MHz) δ: 2.0-2.2 (2H, m), 2.5-2.7 (2H, m), 3.2-3.6 (5H, m), 3.66 (1H, t, J=11Hz), 3.82 (3H, s), 4.2-4.3 (1H, m), 5.53 (1H, d, J=3Hz), 6.28 (1H, dd, J=1, 3Hz), 6.64 (1H, br d, J=7Hz), 6.70 (1H, br s), 6.85 (1H, br d, J=8Hz), 6.97 (1H, dd, J=3, 5Hz), 7.02 (1H, d, J=3Hz), 7.19 (1H, dd, J=7, 8Hz), 7.32 (1H, d, J=5Hz), 7.55 (1H, br s).

IR (cm⁻¹, KBr): 3425, 3367, 2956, 1736, 1686, 1678, 1630, 1622, 1603, 1560, 1493, 1470, 1396, 1358, 1298, 1203, 1184, 1138, 997, 951, 798, 762, 721.

### [Example 39]

### [Pharmacological Experiments]

### I. Methods of measurement

### (1) Binding affinity to human µ opioid receptor

The experiments for binding to µ opioid receptor were performed using a membrane specimen (RECEPTOR BIOLOGY INC.) of human µ opioid receptor (GenBank Accession No. L25119) expressed in CHO-Kl cells by incorporation of gene. As a radio-ligand, [³H]DAMGO was employed.

The membrane specimen and [³H] DAMGO (final concentration: 5nM) were incubated at 22°C for 2.5 hours in the presence of a test compound. The reaction was stopped by suction filtration by a cell harvester using GF/B filter, and Tris-HCl buffer solution was used for washing. The radio activity remaining on the membrane was measured by a liquid scintillation counter. The specific binding amount of [³H] DAMGO was calculated in terms of difference between the total binding amount and the binding amount measured in the presence of 100 nM of naloxone.

The binding ratio in the presence of the test compound at each concentration against the specific binding of [³H] DAMGO was calculated to give IC₅₀ value using Graph Pad Prism.

### (2) Analgesic effect (acetic acid-writhing method)

ICR male mice (8 mice for one group) were used. The test compound was subcutaneously injected. After 30 minutes, 0.6% aqueous acetic acid solution (0.1 mL/10 g body weight) was intraperitoneally injected. The writhing number within the following 20 minutes was measured. From the inhibition ratio in comparison with the number of the control group, ED₅₀ was calculated.

### (3) Experiments for antagonism by peripheral and central (systemic) µ-opioid receptor antagonists

ICR male mice (8 mice for one group) were used. Naloxone methiodide (peripheral µ-opioid receptor antagonist which does not pass blood-brain barrier) or Naloxone hydrochloride (systemic µ-opioid receptor antagonist) was intraperitoneally injected (dosage: 5 mg/kg). After 10 minutes, the test compound was subcutaneously injected. Then, after 20 minutes, 0.6% aqueous acetic acid solution (0.1 mL/10 g body weight) was intraperitoneally injected. The writhing number within the following 20 minutes was measured. Comparison was made between the writhing inhibition ratio observed on the group administered with the test compound only and the writhing inhibition ratio observed on the group which was pre-treated with Naloxone methiodide or Naloxone hydrochloride.

### II. Test results

### [Test results]

### (1) Binding assay to µ-receptor

**Table 22**

| Test compound | IC₅₀ (nM) |
|---|---|
| Fentanyl | 21 |
| Comparison compound X | 44 |
| Example 12 | 100 |
| Example 15 | 108 |
| Example 17 | 27 |
| Example 19 | 56 |
| Example 21 | 25 |
| Example 25 | 60 |
| Example 29 | 33 |
| Example 32 | 57 |
| Example 36 | 41 |
| Example 38 | 40 |

Example numbers indicate the compound obtained in the aforementioned synthesis Examples (same for Tables 23 and 24) .

Comparison compound X: 3-[4-methoxycarbonyl-4-(phenylpropionylamino)piperidin-1-yl]-2-phenylpropionic acid (refer to Reference Example 2 of Patent publication 5, the same for Table 24)

### (2) Analgesic effect

**Table 23**

| Test compound | ED₅₀ (mg/kg, sc) |
|---|---|
| Example 12 | 0.62 |
| Example 15 | 0.72 |
| Example 17 | 0.34 |
| Example 19 | 0.46 |
| Example 21 | 0.15 |
| Example 25 | 0.59 |
| Example 29 | 0.10 |
| Example 32 | 0.78 |
| Example 34 | 0.26 |
| Example 36 | 0.55 |
| Example 38 | 0.66 |

### (3) Experiments for antagonism caused by peripheral or central (systemic) µ-opioid receptor antagonist

**Table 24**

| Test compound | Dosage (mg/kg, sc) | Inhibition ratio(%) of Writhing No. Pre-treatment | | |
|---|---|---|---|---|
| | | None | Peripheral antagonist | Systemic antagonist |
| Fentanyl | 0.05 | 100 | 99 | -21 |
| Comparison X | 3.0 | 100 | 100 | 5 |
| Example 12 | 1.0 | 64 | 20 | 4 |
| Example 15 | 2.0 | 50 | 6 | |
| Example 17 | 1.5 | 83 | 11 | |
| Example 19 | 1.4 | 81 | 21 | |
| Example 27 | 2.0 | 73 | 10 | |
| Example 32 | 2.3 | 69 | 13 | |

The results in Tables 22 and 23 clearly indicate that the invention compounds show excellent binding affinity to peripheral µ-receptor and excellent analgesic effect. Further, the results in Table 24 clearly indicate that the µ-opioid receptor antagonist reverses the analgesic effects of the invention compounds (that is, the analgesic effect almost disappears).

In contrast, the analgesic effect of Comparison compound X (as well as the analgesic effect of Fentanyl) is not affected by the pre-treatment with the peripheral µ-opioid receptor antagonist but completely disappears in the case that the pre-pretreatment with the systemic µ-opioid receptor antagonist. Accordingly, it was confirmed that the analgesic effect of Comparison compound X does not mediate the peripheral µ-opioid receptor, but mediate the central µ-opioid receptor.

Thus, the above-mentioned results indicate that the invention compounds have an analgesic effect mediate the peripheral µ-opioid receptor, while Comparison compound X shows an analgesic effect mediated the central µ-opioid receptor. Thus, the mechanism of the analgesic effect is clearly different from each other. In addition, it is expected that the invention compounds do not show adverse effects such as drug dependence and drowsiness which mediated the central opioid receptors.

## Claims

1. Compounds having the following formula (I) or salts thereof: 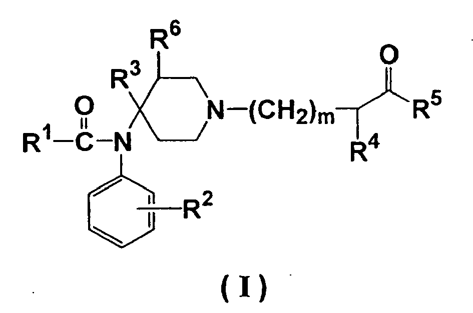 in which
R¹ represents an alkyl group having 1 to 6 carbon atoms, a cycloalkyl group having 3 to 8 ring-forming atoms, an alkyl group having 1 to 6 carbon atoms which has an alkoxy substituent having 1 to 6 carbon atoms, or a heterocyclic group having 5 or 6 ring-forming atoms which optionally has 1 to 3 substituents selected from the group consisting of halogen atoms and alkyl groups having 1 to 6 carbon atoms;
R² represents an amino group, an alkylamino group having 1 to 6 carbon atoms, a dialkylamino group having 2 to 12 carbon atoms, an acylamio group having 1 to 6 carbon atoms, an alkyl(thiocarbonyl)amino group having 2 to 6 carbon atoms, a ureido group, a thioureido group, a guanidino group, an alkoxycarbonylamino group having 2 to 6 carbon atoms, or an alkylsulfonylamino group having 1 to 6 carbon atoms, provided that R² is placed in ortho or meta position;
R³ represents a hydrogen atom, an alkoxycarbonyl group having 2 to 8 carbon atoms, or a methyl group having an alkoxy substituent having 1 to 6 carbon atoms;
R⁴ represents a phenyl or thienyl group which optionally has 1 to 3 substituents selected from the group consisting of a halogen atom, an alkyl group having 1 to 6 carbon atoms, an alkoxy group having 1 to 6 carbon atoms, an alkyl group having 1 to 6 carbon atoms which is substituted with a halogen atom, a nitro group, a cyano group, and an amino group;
R⁵ represents OR⁷ or NR⁸R⁹ in which each of R⁷, R⁸ and R⁹ is a hydrogen atom, an alkyl group having 1 to 6 carbon atoms, or an aralkyl group having an alkyl moiety of 1 to 6 carbon atoms and an aryl moiety which optionally has a substituent selected from the group consisting of an alkyl group having 1 to 6 carbon atoms and an alkoxy group having 1 to 6 carbon atoms;
R⁶ represents a hydrogen atom or a methyl group;
and
m represents 1 or 2.

2. The compound or a salt thereof defined in claim 1, in which R⁵ represents OR⁷ in which R⁷ is a hydrogen atom, an alkyl group having 1 to 6 carbon atoms, or an aralkyl group having an alkyl moiety of 1 to 6 carbon atoms and an aryl moiety which optionally has a substituent selected from the group consisting of an alkyl group having 1 to 6 carbon atoms and an alkoxy group having 1 to 6 carbon atoms.

3. The compound or a salt thereof defined in claim 1, in which R⁵ is a hydroxyl group.

4. The compound or a salt thereof defined in any one of claims 1 to 3, in which R⁶ is a hydrogen atom.

5. The compound or a salt thereof defined in any one of claims 1 to 4, in which m is 1.

6. The compound or a salt thereof defined in any one of claims 1 to 5, in which R² is an amino group, an acetylamino group, a ureido group, or a guanidino group.

7. The compound or a salt thereof defined in any one of claims 1 to 6, in which R² is placed in the meta position.

8. The compound or a salt thereof defined in any one of claims 1 to 7, in which R³ is a hydrogen atom or an alkoxycarbonyl group having 2 to 8 carbon atoms.

9. The compound or a salt thereof defined in any one of claims 1 to 7, in which R³ is a hydrogen atom.

10. The compound or a salt thereof defined in any one of claims 1 to 7, in which R³ is an alkoxycarbonyl group having 2 to 8 carbon atoms.

11. The compound or a salt thereof defined in any one of claims 1 to 10, in which R¹ is an alkyl group having 1 to 6 carbon atoms or a heterocyclic group having 5 or 6 ring-forming atoms which optionally has 1 to 3 substituents selected from the group consisting of halogen atoms and alkyl groups having 1 to 6 carbon atoms.

12. The compound or a salt thereof defined in any one of claims 1 to 10, in which R¹ is an alkyl group having 1 to 6 carbon atoms.

13. The compound or a salt thereof defined in any one of claims 1 to 10, in which R¹ is a heterocyclic group having 5 or 6 ring-forming atoms which optionally has 1 to 3 substituents selected from the group consisting of halogen atoms and alkyl groups having 1 to 6 carbon atoms.

14. The compound or a salt thereof defined in any one of claims 1 to 10, in which R¹ is a furyl group or a thienyl group.

15. The compound or a salt thereof defined in any one of claims 1 to 10, in which R¹ is a furyl group.

16. The compound or a salt thereof defined in any one of claims 1 to 15, in which R⁴ is a phenyl group.

17. The compound or a salt thereof defined in any one of claims 1 to 15, in which R⁴ is a thienyl group.

18. A compound or a salt thereof, the compound being selected from the group consisting of:
3-[4-[N-(3-guanidinophenyl)-N-propionylamino]-piperidin-1-yl]-2-phenylpropionic acid,
3-[4-[N-(3-aminophenyl)-N-(2-furoyl)amino]-4-methoxycarbonylpiperidin-1-yl]-2-phenylpropionic acid,
3-[4-[N-(2-furoyl)-N-(3-ureidophenyl)amino]-4-methoxycarbonylpiperidin-1-yl]-2-phenylpropionic acid,
3-[4-[N-(3-acetylaminophenyl)-N-(2-furoyl)amino]-4-methoxycarbonylpiperidin-1-yl]-2-phenylpropionic acid,
3-[4-[N-(3-acetylaminophenyl)-N-propionylamino]-4-methoxycarbonylpiperidin-1-yl]-2-phenylpropionic acid,
and
cis-3-[4-[N-(2-furoyl)-N-(3-ureidophenyl)amino]-3-methylpiperidin-1-yl]-2-phenylpropionic acid.

19. An analgesic containing an compound or a salt thereof defined in any one of claims 1 to 18 as an effective ingredient.

20. The analgesic defined in claim 19, in which an analgesic effect is peripheral.
